(19) Europäisches Patentamt
European Patent Office
Office européen des brevets

(11) EP 2 941 415 B1

(12) **FASCICULE DE BREVET EUROPEEN**

(45) Date de publication et mention de la délivrance du brevet:
**26.04.2017 Bulletin 2017/17**

(21) Numéro de dépôt: **14701812.1**

(22) Date de dépôt: **07.01.2014**

(51) Int Cl.:
***C07C 253/30*** *(2006.01)*

(86) Numéro de dépôt international:
**PCT/FR2014/050012**

(87) Numéro de publication internationale:
**WO 2014/106724 (10.07.2014 Gazette 2014/28)**

(54) **PROCÉDÉ DE MÉTATHÈSE CROISÉE**

KREUZMETATHESEVERFAHREN

CROSS METATHESIS PROCESS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.01.2013 FR 1350103**

(43) Date de publication de la demande:
**11.11.2015 Bulletin 2015/46**

(73) Titulaire: **Arkema France**
**92700 Colombes (FR)**

(72) Inventeurs:
- **DUBOIS, Jean-Luc**
**F-69390 Millery (FR)**
- **COUTURIER, Jean-Luc**
**F-69006 Lyon (FR)**

(74) Mandataire: **Hérard, Elise et al**
**Arkema France**
**DRD - DPI**
**420 rue d'Estienne d'Orves**
**92705 Colombes Cedex (FR)**

(56) Documents cités:
**WO-A1-2011/138051 WO-A2-2008/104722**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 941 415 B1

Printed by Jouve, 75001 PARIS (FR)

## Description

### DOMAINE DE L'INVENTION

La présente invention concerne un procédé de métathèse croisée, pour la production d'un produit insaturé tel qu'un nitrile-ester ou un nitrile-acide insaturé.

### ARRIERE-PLAN TECHNIQUE

L'industrie des polyamides utilise toute une gamme de monomères formés à partir de diamines et de diacides, de lactames, et surtout à partir d'ω-aminoacides. Ces derniers sont définis par la longueur de chaîne méthylène $(-CH_2)_n$ séparant deux fonctions amide -CO-NH-. Ces monomères sont classiquement fabriqués par voie de synthèse chimique en utilisant comme matières premières des oléfines en C2 à C4, des cycloalcanes ou du benzène, hydrocarbures issus de sources fossiles. Par exemple, les oléfines en C2 servent à fabriquer l'aminoacide en C9 utilisé dans le Pelargon russe ; les oléfines en C4 servent à fabriquer l'héxaméthylène diamine ; le laurolactame et le caprolactame sont fabriqués à partir de cycloalcanes ; l'acide adipique, le Nylon 6 et le Nylon 6,6 sont fabriqués à partir de benzène.

L'évolution actuelle en matière d'environnement conduit dans les domaines de l'énergie et de la chimie à privilégier l'exploitation des matières premières naturelles provenant d'une source renouvelable. C'est la raison pour laquelle certains travaux ont été entrepris pour élaborer sur le plan industriel des procédés utilisant des acides/esters gras comme matière première de fabrication de ces monomères.

Dans le document FR 2912741 est ainsi décrit un procédé de synthèse de toute une gamme d'aminoacides/esters à partir d'un acide/ester gras naturel à longue chaîne, en soumettant ce dernier à une réaction de métathèse catalytique croisée avec un composé insaturé comportant une fonction nitrile, suivie d'une hydrogénation.

Dans le document FR 2938533 est décrit un procédé de synthèse d'acides ω-amino-alcanoïques ou de leurs esters à partir d'acides gras naturels insaturés à longue chaîne, transitant par un composé intermédiaire de type nitrile ω-insaturé dont l'une des variantes met en oeuvre dans la phase finale une métathèse croisée du nitrile ω-insaturé avec un composé de type acrylate.

Dans le document FR 2941694 est décrite une variante du procédé précédent dans laquelle le composé intermédiaire est du type dinitrile insaturé.

Ces procédés conduisent, à l'issue d'une étape d'hydrogénation de la fonction nitrile et de la double-liaison, à la fabrication d'aminoacides.

Enfin, le document FR 2959742 a pour objet d'améliorer les performances des procédés mettant en oeuvre successivement une métathèse croisée et une hydrogénation.

Dans ces procédés, les réactions de métathèse croisée, généralement effectuées entre un nitrile gras oméga insaturé et un acrylate, ou entre un ester gras oméga insaturé et l'acrylonitrile, conduisent non seulement au produit désiré qui est un nitrile-ester, mais aussi à des produits issus de réaction d'homométathèse des corps gras, tels que respectivement des dinitriles et des diesters. En augmentant les quantités de catalyseur mises en oeuvre, les temps de réaction, et/ou les ratios entre les réactifs, il est possible de convertir ces coproduits issus de l'homométathèse en nitrile-ester, mais ces solutions s'avèrent couteuses et peu productives.

D'autre part, les produits des réactions d'homométathèse (diesters ou dinitriles) sont des produits lourds, à longue chaine, qui ont des applications limitées, et souvent déconnectées des applications industrielles recherchées pour les nitriles esters.

Il existe donc un réel besoin de mettre au point un procédé de synthèse d'un composé gras insaturé par métathèse croisée (et notamment de synthèse de nitrile-ester/acide) dans lequel la quantité de coproduits issus des réactions d'homométathèse est réduite et ne nécessitant pas de consommation excessive de catalyseur.

### RESUME DE L'INVENTION

L'invention concerne en premier lieu un procédé de synthèse d'un produit insaturé par métathèse croisée entre un premier composé insaturé comportant au moins 8 atomes de carbone et un deuxième composé insaturé comportant moins de 8 atomes de carbone, le premier composé insaturé étant susceptible de produire un coproduit insaturé, comportant plus de 14 atomes de carbone, par homométathèse, ledit procédé comprenant au moins une phase de production qui comprend :

- l'alimentation d'un réacteur avec le premier composé insaturé ;
- l'alimentation du réacteur avec le deuxième composé insaturé ;
- l'alimentation du réacteur avec au moins un premier catalyseur de métathèse suivie par l'alimentation du réacteur avec au moins un deuxième catalyseur de métathèse ;

- le prélèvement d'un flux produit issu du réacteur ;

le nombre de rotations du premier catalyseur étant supérieur au nombre de rotations du deuxième catalyseur pour atteindre un même taux de conversion cible du premier composé insaturé, les nombres de rotations des catalyseurs étant déterminés dans des conditions de température, de pression, de stoechiométrie, et de concentrations de réactifs identiques à celles de la phase de production.

Selon un mode de réalisation, le taux de conversion cible ($TTG_C$) vaut de 30 % à 95 %, de préférence de 40 % à 90 %, notamment de 50 à 80 %, plus particulièrement de 60 à 75 %.

Selon un mode de réalisation, la phase de production est semi-continue, et comprend de préférence successivement :

(1) l'alimentation du réacteur avec la totalité du premier composé insaturé et une première partie du deuxième composé insaturé, avant le démarrage de la réaction ;
(2) l'alimentation progressive du réacteur avec une deuxième partie du deuxième composé insaturé et avec le premier catalyseur ;
(3) l'alimentation progressive du réacteur avec une troisième partie du deuxième composé insaturé et avec le deuxième catalyseur.

Selon un mode de réalisation :

- le premier composé insaturé est de formule :

(I) $R_1\text{-CH=CH-}(CH_2)_n\text{-}R_2$ ;

- le deuxième composé insaturé est de formule :

(II) $R_3\text{-CH=CH-}R_4$ ;

- le produit insaturé est de formule :

(III) $R_4\text{-CH=CH-}(CH_2)_n\text{-}R_2$ ;

- le coproduit insaturé est de formule :

(IV) $R_2\text{-}(CH_2)_n\text{-CH=CH-}(CH_2)_n\text{-}R_2$ ;

$R_1$ représentant un atome d'hydrogène ou un radical alkyle ou alkényle comportant de 1 à 8 atomes de carbone ; $R_2$ représentant $COOR_5$ ou CN ou CHO ou $CH_2OH$ ou $CH_2Cl$ ou $CH_2Br$ ; $R_3$ et $R_4$ représentant chacun un atome d'hydrogène ou un radical alkyle comportant de 1 à 4 atomes de carbone ou $COOR_5$ ou CN ou CHO ou $CH_2OH$ ou $CH_2Cl$ ou $CH_2Br$, $R_3$ et $R_4$ étant identiques ou différents et ne comportant au total pas plus de 6 atomes de carbone ; $R_5$ représentant un atome d'hydrogène ou un radical alkyle comportant de 1 à 4 atomes de carbone ; et n étant un nombre entier de 4 à 11.

Selon un mode de réalisation, le deuxième composé insaturé est un acrylate ou de préférence l'acrylonitrile, le premier composé insaturé est un acide, un nitrile insaturé ou un ester insaturé, de préférence choisi parmi le 9-décénoate de méthyle, le 9-décènenitrile, le 10-undécènenitrile et le 10-undécénoate de méthyle, le produit insaturé est un nitrile-ester, un nitrile-acide, un dinitrile ou un diester insaturé, et le coproduit insaturé est un diester, un dinitrile ou un diacide insaturé.

Selon un mode de réalisation, les réactions de métathèse sont effectuées en phase liquide, le cas échéant dans un solvant, et résultent de préférence en la production d'au moins un composé insaturé sous forme gazeuse, de manière plus particulièrement préférée l'éthylène, dans le réacteur, le procédé comprenant le soutirage de celui-ci du réacteur de manière continue.

Selon un mode de réalisation, le taux de conversion du premier composé insaturé à l'issue de la phase de production est de 50 à 98 %, de préférence de 60 à 95 %, de manière plus particulièrement préférée de 70 à 90 %.

Selon un mode de réalisation, le procédé comprend une phase préliminaire de sélection du premier catalyseur et du deuxième catalyseur parmi un ensemble de catalyseurs possibles, avant la phase de production.

Selon un mode de réalisation, la phase préliminaire de sélection comprend :

- la mise en oeuvre d'un procédé de référence pour chaque catalyseur possible, chaque procédé de référence comprenant l'alimentation du réacteur avec le premier composé insaturé, l'alimentation du réacteur avec le deuxième composé insaturé et l'alimentation du réacteur avec le catalyseur, les conditions de température, de pression, de stoechiométrie et de concentrations de réactifs pour chaque procédé de référence étant identiques à celles utilisées pour la phase de production ;
- la collecte des résultats des procédés de référence, comprenant la mesure de concentrations d'espèces chimiques dans le réacteur au cours du temps ;
- le choix du premier catalyseur et du deuxième catalyseur en fonction des résultats des procédés de référence.

Selon un mode de réalisation, le premier catalyseur est le catalyseur présentant le nombre de rotations maximal dans le procédé de référence, pour un même taux de conversion cible du premier composé insaturé.

Selon un mode de réalisation, le deuxième catalyseur est un catalyseur pour lequel, dans le procédé de référence, la valeur absolue de la dérivée du rendement de la réaction en coproduit insaturé en fonction du taux de conversion du premier composé insaturé, déterminée au taux de conversion de 1, est minimale.

Selon un mode de réalisation, le deuxième catalyseur est choisi, parmi les catalyseurs pour lesquels, dans le procédé de référence, la valeur absolue de la dérivée du rendement de la réaction en coproduit insaturé en fonction du taux de conversion du premier composé insaturé mesurée au taux de conversion de 1 est minimale, comme étant le catalyseur pour lequel le nombre de rotations dans le procédé de référence est maximal, pour un même taux de conversion cible du premier composé insaturé.

Selon un mode de réalisation :

- on calcule à différents instants de chaque procédé de référence le nombre X qui est défini par la formule suivante :

$$(1) \qquad X=(TTU_2/2)\times(z/C_0-TTU_1)/((1-TTG)\times TTU_1),$$

  dans laquelle $C_0$ représente le nombre de moles du premier composé insaturé dans le réacteur avant le début de l'alimentation en catalyseur, TTG représente le taux de conversion du premier composé insaturé à l'instant considéré, $TTU_1$ représente le rendement de la réaction en produit insaturé à l'instant considéré, $TTU_2$ représente le rendement de la réaction en coproduit insaturé à l'instant considéré, et z représente le nombre total cumulé de moles de deuxième composé insaturé introduites dans le réacteur à l'instant considéré ;
- on calcule à différents instants de chaque procédé de référence le nombre Y qui est défini par la formule suivante :

$$(2) \qquad Y=(p\times\alpha/(2+p))\times(z/(C_0\times TTU_1)-1) + (2+2p)\times(1-TTG)/((2+p)\times TTU_1)$$

  dans laquelle $C_0$, TTG, $TTU_1$ et z ont la signification ci-dessus, p représente la valeur absolue (donc valeur positive) de la dérivée de la fonction $TTU_2(TTG)$ pour un taux de conversion égal à 1, et $\alpha$ vaut $(2a/b)\times(C_0/C'_0)$, a représentant la dérivée de la fonction $TTU_1(TTG)$ pour un taux de conversion égal à 0, b représentant la dérivée de la fonction $TTU_2(TTG)$ pour un taux de conversion égal à 0 et $C'_0$ représentant le nombre de moles du deuxième composé insaturé dans le réacteur avant le début de l'alimentation en catalyseur ;
- on approxime la fonction Y(X) par une équation $Y=\beta\times X$ ou, de manière préférée, par une équation $Y=\beta'\times X-\gamma'$, pour les données expérimentales obtenues pour les conversions les plus élevées, c'est-à-dire celles obtenues au-delà de la conversion correspondant au maximum de rendement en coproduit insaturé ($\beta$, $\beta'$ et $\gamma'$ étant nécessairement des valeurs positives) ;
- on sélectionne le deuxième catalyseur comme étant le catalyseur pour lequel le paramètre $\beta$ ou $\beta'$ est maximal.

Selon un mode de réalisation, le réacteur est alimenté en premier catalyseur jusqu'à ce que le taux de conversion du premier composé insaturé atteigne une valeur seuil, ladite valeur seuil étant de 30 à 90 %, de préférence de 40 à 80 %, et plus particulièrement de 50 à 70 %.

Selon un mode de réalisation, le réacteur est alimenté en premier catalyseur pendant une durée égale, ou de préférence supérieure, à une durée seuil, ladite durée seuil étant celle au bout de laquelle le taux de conversion du premier composé insaturé vaut $TTG=TTG_S$ et le rendement en coproduit insaturé vaut $TTU_2=TTU_{2S}$ dans un procédé de référence, sont reliés par l'équation $TTU_{2S}=TTU^*\times(TTG_S-1)/(TTG^*-1)$, où $TTU^*$ est la valeur du rendement en coproduit insaturé souhaité à l'issue de la phase de production, et $TTG^*$ est la valeur du taux de conversion du premier composé insaturé souhaité à l'issue de la phase de production, le procédé de référence comprenant l'alimentation du

réacteur avec le premier composé insaturé, l'alimentation du réacteur avec le deuxième composé insaturé et l'alimentation du réacteur avec le premier catalyseur, les conditions de température, de pression, de stoechiométrie et de concentrations de réactifs pour le procédé de référence étant identiques à celles utilisées pendant la phase de production.

Selon un mode de réalisation, le réacteur est alimenté en premier catalyseur pendant une durée égale, ou de préférence supérieure, à une durée seuil qui est la durée au bout de laquelle, dans un procédé de référence, la dérivée seconde du nombre de rotations du premier catalyseur en fonction du temps s'annule, le procédé de référence comprenant l'alimentation du réacteur avec le premier composé insaturé, l'alimentation du réacteur avec le deuxième composé insaturé et l'alimentation du réacteur avec le premier catalyseur, les conditions de température, de pression, de stoechiométrie et de concentrations de réactifs pour le procédé de référence étant identiques à celles utilisées pendant la phase de production.

Selon un mode de réalisation, le procédé comprend, avant la phase de production, une phase préliminaire de détermination de la durée d'introduction du premier catalyseur, qui comprend :

- la mise en oeuvre du procédé de référence ;
- la collecte des résultats du procédé de référence, comprenant la mesure de concentrations d'espèces chimiques dans le réacteur au cours du temps ; et
- le calcul de la durée d'introduction du premier catalyseur en fonction des résultats des procédés de référence.

Selon un mode de réalisation, le premier catalyseur et / ou le deuxième catalyseur sont des catalyseurs ruthénium-carbène, et de préférence sont choisis parmi les catalyseurs de formule (A) et (B) suivantes :

(A)

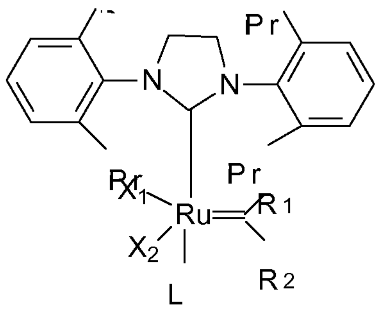

(B)

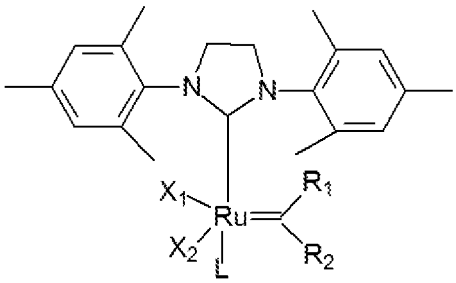

dans lesquelles $X_1$ et $X_2$ sont des ligands anioniques, L est un ligand neutre électrodonneur, et $R_1$ et $R_2$ représentent H ou un substituant contenant de 1 à 20 atomes de carbone de type alkyle, alkenyle, alkynyle, aryle, alcoxy, alkenyloxy, alkynyloxy, aryloxy, alcoxycarbonyle, alkylthiol, arylthiol, alkylsulfonyle, alkylsulfinyle ; le substituant contenant éventuellement des groupements de type hydroxyle, thiol, thioéther, cétone, aldéhyde, ester, éther, amine, imine, amide, nitro, acide carboxylique, disulfure carbonate, isocyanate, carbodiimide, carboalcoxy, carbamate ou halogène, $R_2$ étant de préférence lié à L pour former un ligand chélatant.

Selon un mode de réalisation, le premier catalyseur et / ou le deuxième catalyseur sont choisis parmi les catalyseurs de formules (A-1) à (A-10) et (B-1) à (B-5) suivantes :

(A-1)

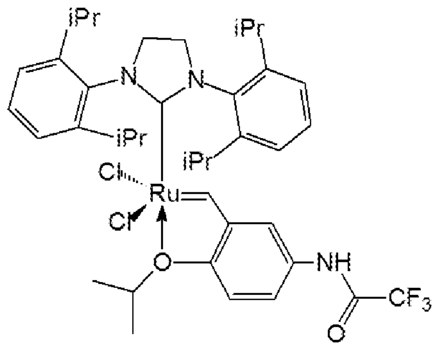

(A-2)

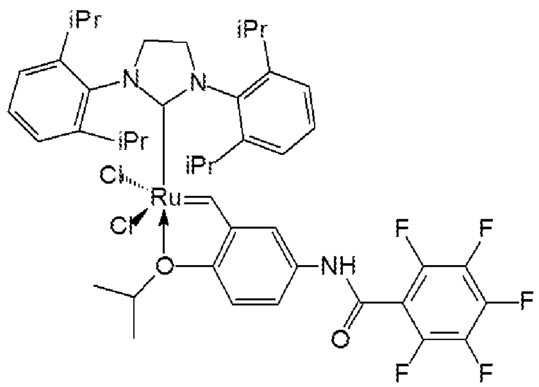

(A-3)

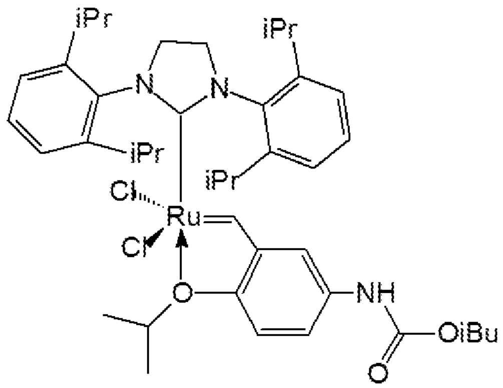

(A-4)

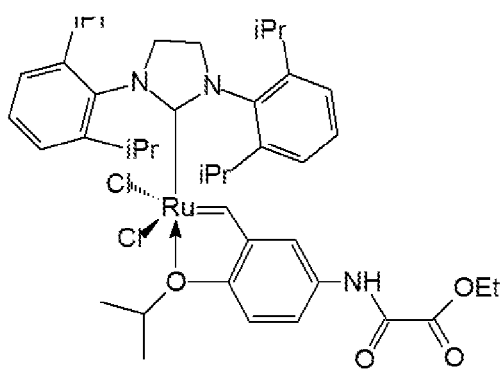

(A-5)

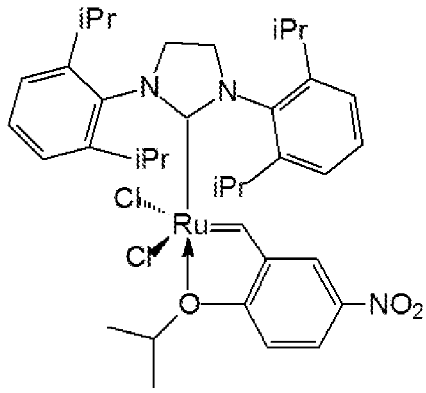

(A-6)

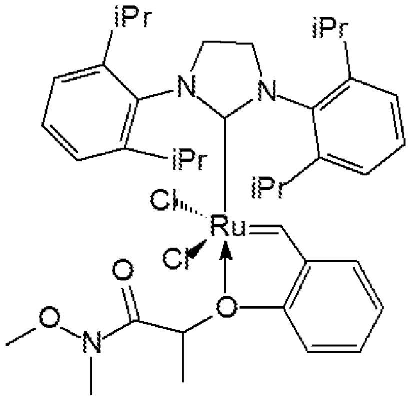

(A-7)

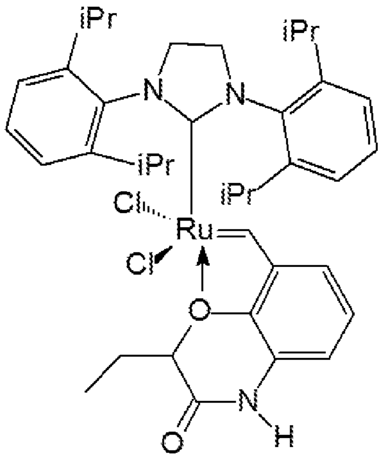

(A-8)

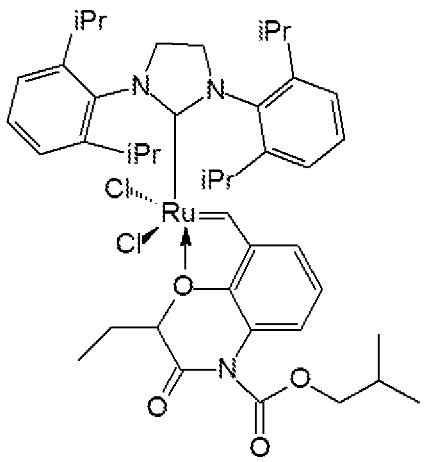

(A-9)

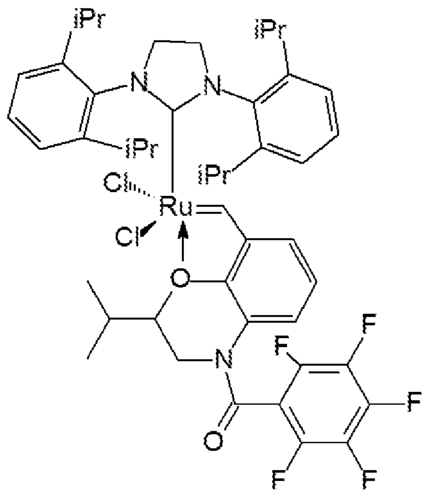

(A-10)

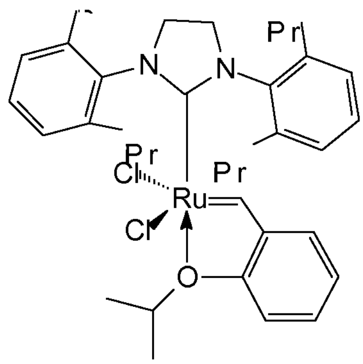

(B-1)

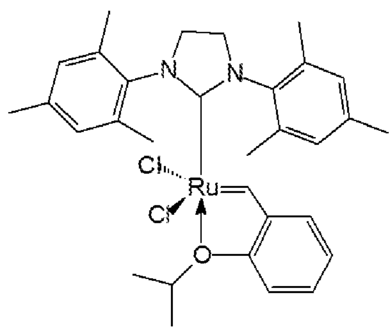

(B-2)

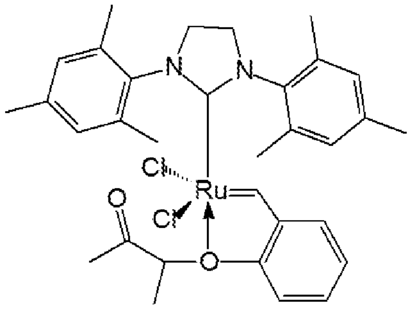

(B-3)

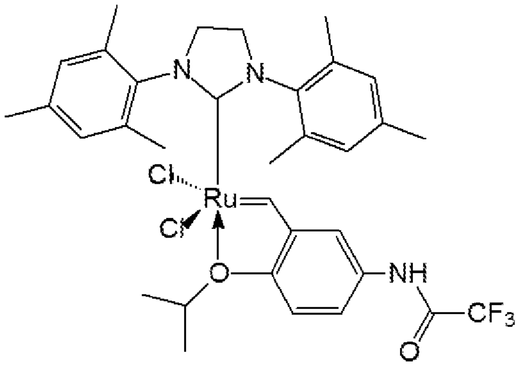

(B-4)

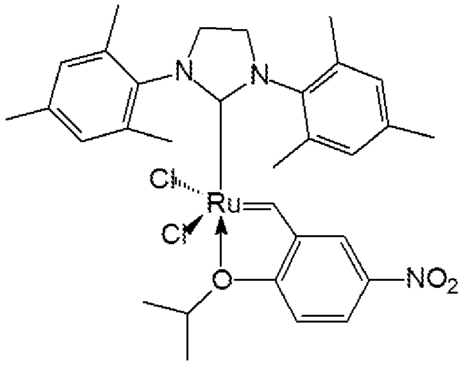

(B-5)

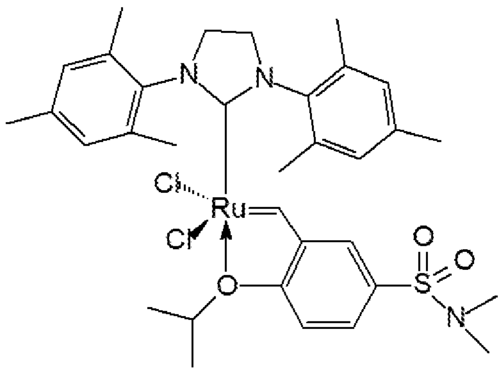

Selon un mode de réalisation, le premier catalyseur est un catalyseur de formule (A) et le deuxième catalyseur est un catalyseur de formule (B), ou de préférence le premier catalyseur est choisi parmi les catalyseurs de formules (A-1) à (A-10) et le deuxième catalyseur est choisi parmi les catalyseurs de formules (B-1) à (B-5).

L'invention concerne également un procédé de synthèse d'un acide ou d'un ester $\alpha,\omega$-aminoalcanoïque, comprenant la synthèse d'un produit insaturé selon le procédé ci-dessus, qui est un nitrile-ester ou un nitrile-acide insaturé, et une réaction d'hydrogénation de celui-ci.

La présente invention permet de surmonter les inconvénients de l'état de la technique. Elle fournit plus particulièrement un procédé de synthèse d'un composé gras insaturé par métathèse croisée (et notamment de synthèse de

nitrile-ester/acide) dans lequel la quantité de coproduits issus des réactions d'homométathèse est réduite et la quantité de catalyseur consommée est également réduite.

L'invention repose sur la découverte que l'utilisation d'au moins deux catalyseurs différents, dans un ordre particulier, permet de minimiser le rendement en coproduit. Les performances de la réaction sont ainsi meilleures qu'avec un seul catalyseur ou avec un mélange de catalyseurs.

BREVE DESCRIPTION DES FIGURES

La **figure 1** est une illustration des résultats collectés lors d'un procédé de référence utilisé dans le cadre de l'invention. Le taux de conversion du premier composé insaturé figure en abscisse et le rendement en produit insaturé ($TTU_1$, points o) et en coproduit insaturé ($TTU_2$, points □) figurent en ordonnée. On sélectionne parmi les données recueillies pour cette figure le taux de conversion cible $TTG_C$ pour lequel on effectue la comparaison avec d'autres catalyseurs.

La **figure 2** est une illustration d'une méthode de détermination graphique d'une durée seuil $TS_1$ pour l'introduction du premier catalyseur dans le réacteur (à partir des résultats de la **figure 1**). Sont reportés sur la figure le point correspondant au taux de conversion visé à la fin du cycle de production, et le rendement en coproduit visé, respectivement TTG* et $TTU_2$*. Le point d'intersection avec la courbe du rendement $TTU_2$(TTG) correspondant à [$TTG_S$ ; $TTU_{2S}$] donne aussi le temps de seuil $T_{S1}$ correspondant au temps à partir duquel on peut procéder à la permutation des catalyseurs.

La **figure 3** est un graphique montrant le rendement en produit insaturé ($TTU_1$, points o) et en coproduit insaturé ($TTU_2$, points □) (en ordonnée) en fonction du taux de conversion du premier composé insaturé (en abscisse), dans l'expérience de l'exemple 1 ci-dessous.

La **figure 4** est un graphique montrant le rendement en produit insaturé ($TTU_1$, points o) et en coproduit insaturé ($TTU_2$, points □) (en ordonnée) en fonction du taux de conversion du premier composé insaturé (en abscisse), dans l'expérience de l'exemple 2 ci-dessous.

La **figure 5** est un graphique montrant le rendement en produit insaturé ($TTU_1$, points o) et en coproduit insaturé ($TTU_2$, points □) (en ordonnée) en fonction du taux de conversion du premier composé insaturé (en abscisse), dans l'expérience de l'exemple 3 ci-dessous.

La **figure 6** est un graphique montrant le rendement en produit insaturé ($TTU_1$, points o) et en coproduit insaturé ($TTU_2$, points □) (en ordonnée) en fonction du taux de conversion du premier composé insaturé (en abscisse), dans l'expérience de l'exemple 4 ci-dessous.

La **figure 7** est un graphique montrant le rendement en produit insaturé ($TTU_1$, points o) et en coproduit insaturé ($TTU_2$, points o) (en ordonnée) en fonction du taux de conversion du premier composé insaturé (en abscisse), dans l'expérience de l'exemple 5 ci-dessous.

DESCRIPTION DE MODES DE REALISATION DE L'INVENTION

L'invention est maintenant décrite plus en détail et de façon non limitative dans la description qui suit.

Réaction de métathèse

L'invention met en oeuvre une réaction de métathèse entre un composé gras insaturé comportant au moins 8 atomes de carbone, appelé premier composé insaturé, et une oléfine fonctionnelle ou non comportant moins de 8 atomes de carbone, appelée deuxième composé insaturé.

Le premier composé insaturé a pour formule (I) $R_1$-CH=CH-$(CH_2)_n$-$R_2$ et le deuxième composé insaturé a pour formule (II) $R_3$-CH=CH-$R_4$ avec :

- $R_1$=H ou radical alkyle ou alkényle comportant de 1 à 8 atomes de carbone ;
- $R_2$=$COOR_5$, CN, CHO, $CH_2OH$, $CH_2Cl$ ou $CH_2Br$ ;
- $R_3$ et $R_4$=H, radical alkyle de 1 à 4 atomes de carbone, $COOR_5$, CN, CHO, $CH_2OH$, $CH_2Cl$ ou $CH_2Br$, $R_3$ et $R_4$ étant identiques ou différents et $R_3$+$R_4$ ne comprennent pas plus de 6 atomes de carbone ;
- $R_5$=H ou radical alkyle de 1 à 4 atomes de carbone ;
- n est compris entre 4 et 11.

Les réactions mises en jeu sont :

- la métathèse croisée entre les composés (I) et (II) donnant le « produit insaturé » souhaité de formule (III) $R_4$-CH=CH-$(CH_2)_n$-$R_2$, et le composé de formule (V) $R_1$-CH=CH-$R_3$ ;
- une autre métathèse croisée entre les composés (I) et (II) donnant le composé de formule (VI) $R_1$-CH=CH-$R_4$, et le composé de formule (VII) $R_3$-CH=CH-$(CH_2)_n$-$R_2$ ;
- l'homométathèse du composé (I), donnant le « coproduit insaturé » non souhaité (ou souhaité en plus faible quantité), de formule (IV) $R_2$-$(CH_2)_n$-CH=CH-$(CH_2)_n$-$R_2$, ainsi que le composé de formule (VIII) $R_1$-CH=CH-$R_1$ ;
- l'homométathèse croisée du coproduit insaturé de formule (IV) avec le deuxième composé insaturé de formule (II), donnant le produit insaturé souhaité de formule (III) et redonnant le premier composé insaturé de formule (I).

Il n'y a en général pas d'homométathèse détectable du composé de formule (II) lorsque l'on utilise l'acrylonitrile, ou les acrylates.

Dans un mode de réalisation préféré, le procédé implique la formation d'un produit léger éliminable du milieu réactionnel par distillation, ce qui permet de déplacer les équilibres vers la formation des produits souhaités.

Dans ce qui précède, ces composés légers sont les composés de formules $R_1$-CH=CH-$R_3$ et $R_1$-CH=CH-$R_1$.

De manière préférée, le premier composé insaturé est un acide, un nitrile ou un ester et le deuxième composé insaturé est l'acrylonitrile $CH_2$=CH-CN ou un acrylate (par exemple un acrylate de méthyle ou de butyle), le produit insaturé est un nitrile-acide, un ester-acide, un diester, un dinitrile ou un nitrile-ester, et le coproduit insaturé est un diacide, un dinitrile ou un diester.

Le premier composé insaturé peut par exemple être un ester de formule $CH_2$=CH-$(CH_2)_n$-$COOCH_3$, le deuxième composé insaturé étant l'acrylonitrile $CH_2$=CH-CN, auquel cas le produit insaturé est le composé NC-CH=CH-$(CH_2)_n$-$COOCH_3$, et le coproduit insaturé est le diester $CH_3OOC$-$(CH_2)_n$-CH=CH-$(CH_2)_n$-$COOCH_3$. De l'éthylène $CH_2$=$CH_2$ est également produit aussi bien par la métathèse croisée que par l'homométathèse. C'est cet exemple qui est retenu pour illustrer la suite de la description ci-dessous.

Outre les réactions des esters gras avec l'acrylonitrile, d'autres réactions préférées sont celles des nitriles gras avec un acrylate, des esters gras avec un acrylate, des nitriles gras avec l'acrylonitrile, des ester gras avec une oléfine linéaire, des esters gras avec une oléfine ramifiée, des nitriles gras avec une oléfine linéaire, et des nitriles gras avec une oléfine ramifiée.

Le produit insaturé obtenu grâce au procédé selon l'invention peut subir une hydrogénation ultérieure, de manière connue en soi.

<u>Catalyseurs</u>

L'invention repose sur la mise en oeuvre de deux catalyseurs successifs.

Il existe de nombreux catalyseurs pour les réactions de métathèse. On peut citer par exemple les complexes au tungstène développés par Schrock et al (J. Am. Chem. Soc. 108:2771, 1986) ou Basset et al. (Angew. Chem., Ed. Engl. 31:628, 1992). Plus récemment, sont apparus les catalyseurs dits de Grubbs (voir Grubbs et al., Angew. Chem., Ed. Engl. 34 :2039, 1995 et Organic Letters 1:953, 1999) qui sont des complexes ruthénium-benzylidène opérant en catalyse homogène. D'autres travaux ont été conduits pour la réalisation de catalyseurs immobilisés, c'est-à-dire de catalyseurs dont le principe actif est celui du catalyseur homogène, notamment les complexes ruthénium-carbène, immobilisés sur un support inactif.

Le procédé selon l'invention utilise avantageusement un catalyseur de métathèse de type ruthénium-carbène.

Les catalyseurs ruthénium-carbène sont choisis de préférence parmi les catalyseurs chargés ou non-chargés de formule générale :

$$(X_1)_a\ (X_2)_b Ru(\text{carbène C})\ (L_1)_c(L_2)_d(L_3)_e$$

dans laquelle :

- a, b, c, d et e sont des nombres entiers, identiques ou différents, avec a et b égaux à 0, 1 ou 2 ; c,d et e égaux à 0, 1, 2, 3 ou 4;
- $X_1$ et $X_2$, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénolates, les amidures, le tosylate, l'hexafluorophosphate, le tétrafluoroborate, le bis-triflylamidure, un alkyle, le tétraphénylborate et dérivés ; $X_1$ ou $X_2$ peuvent être liés à $L_1$ ou $L_2$ ou au carbène C de façon à former un ligand bidenté ou chélate sur le ruthénium; et
- $L_1$, $L_2$ et $L_3$ identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéther, ou un carbène hétérocyclique ; $L_1$, $L_2$ ou $L_3$ peuvent être

liés au carbène C de façon à former un ligand bidenté ou chélate, ou tridenté.

Le carbène C est représenté par la formule générale : $CR_1R_2$ pour laquelle $R_1$ et $R_2$ sont des groupes identiques ou différents tels que l'hydrogène ou tout autre groupe hydrocarboné, fonctionnalisé ou non, de type saturé, insaturé, cyclique, aromatique, branché et/ou linéaire. A titre d'exemples, on pourra citer les complexes du ruthénium alkylidènes, benzylidène, benzylidène éther, ou cumylènes tels que les vinylidènes Ru=C=CHR ou allénylidènes $Ru=C=C=CR_1R_2$ ou indénylidènes.

Un groupe fonctionnel (permettant d'améliorer la rétention du complexe du ruthénium dans un liquide ionique) peut être greffé sur au moins l'un des ligands $X_1$, $X_2$, $L_1$, $L_2$, ou sur le carbène C. Ce groupe fonctionnel peut être chargé ou non chargé tel que de préférence un ester, un éther, un thiol, un acide, un alcool, une amine, un hétérocycle azoté, un sulfonate, un carboxylate, un ammonium quaternaire, un guanidinium, un phosphonium quaternaire, un pyridinium, un imidazolium, un morpholinium ou un sulfonium.

Le catalyseur de métathèse peut être éventuellement hétérogénéisé sur un support afin de faciliter sa récupération/recyclage.

Les catalyseurs de métathèse croisée du procédé de l'invention sont de préférence des carbènes du ruthénium décrits par exemple dans Aldrichimica Acta, vol 40, n°2, 2007, p.45-52.

Des exemples de tels catalyseurs sont les catalyseurs de Grubbs, les catalyseurs Hoveyda-Grubbs, les catalyseurs Piers-Grubbs, et autres catalyseurs de métathèse du même type, qu'ils soient dits de « 1$^{ère}$ génération », « 2$^{ème}$ génération ».ou de « 3$^{ème}$ génération ».

Les catalyseurs de Grubbs sont basés sur un atome de ruthénium entouré par 5 ligands :

- 2 ligands anioniques, tels que des halogénures ;
- 2 ligands donneurs d'électrons, tels que les tri-alkyl-phosphines, ou les carbènes N-hétérocycliques saturés (appelés ligands NHC) ;
- un groupement alkylidène, tels que des groupements méthylènes $=CR_2$ substitués ou non.

On classe ces catalyseurs de métathèse en deux catégories, suivant la nature de leurs ligands L donneurs d'électrons :

- ceux qui contiennent deux ligands phosphine (et pas de ligand NHC saturé), développés en premier, sont des catalyseurs de type 1$^{ère}$ génération ;
- ceux qui contiennent un ligand NHC saturé (carbène hétérocyclique) sont des catalyseurs de type 2$^{ème}$ génération.

Un type de catalyseur dit « Hoveyda-Grubbs » contient parmi les ligands donneurs d'électrons, un ligand chélatant benzylidène-éther, et soit une phosphine (1$^{ère}$ génération) soit un ligand NHC saturé (2$^{ème}$ génération) généralement substitué par deux mésityls (Mes).

Un autre type de catalyseur dit « Piers-Grubbs », forme un complexe cationique à quatre ligands qui ne nécessite pas la dissociation d'un ligand avant la réaction.

D'autres types de catalyseurs sont les catalyseurs « Umicore », « Zanan », « Grela ».

De manière générale, le choix du catalyseur dépend de la réaction considérée.

Dans un mode de réalisation, le catalyseur est dépourvu de phosphine.

Dans un mode de réalisation, le ligand carbène N-hétérocyclique du catalyseur saturé ou non est substitué par des groupements identiques ou différents constitués de noyaux alkylaromatiques tels que phényle, benzyle, toluyle, mésytile, benzylidène, benzhydryle, diisopropylphényle, mono ou ditertiobutylphényle.

Des catalyseurs préférés sont les catalyseurs suivants de formule (A) ci-dessus (ayant des ligands de type SiPr) et de formule (B) ci-dessus (ayant des ligands de type SiMeS), et notamment de formules (A-1) à (A-10) et (B-1) à (B-5) ci-dessus.

Le catalyseur de formule (A-1) est connu sous le nom « M71-SIPr ». Le catalyseur de formule (A-2) est connu sous le nom « M72-SIPr». Le catalyseur de formule (A-3) est connu sous le nom « M73-SIPr ». Le catalyseur de formule (A-4) est connu sous le nom « M74-SIPr ». Le catalyseur de formule (A-5) est connu sous le nom « Nitro-Grela-SIPr ». Le catalyseur de formule (A-6) est connu sous le nom « Apeiron AS2034 ». Le catalyseur de formule (A-7) est connu sous le nom « M831-SIPr ». Le catalyseur de formule (A-8) est connu sous le nom « M832-SIPr ». Le catalyseur de formule (A-9) est connu sous le nom « M863-SIPr », Le catalyseur de formule (A-10) est connu sous le nom « C711 », Le catalyseur de formule (B-1) est connu sous le nom « Hoveyda-Grubbs 2 ». Le catalyseur de formule (B-2) est connu sous le nom « M51 ». Le catalyseur de formule (B-3) est connu sous le nom « M71-SIMes ». Le catalyseur de formule (B-4) est connu sous le nom « Nitro-Grela-SIMes ». Le catalyseur de formule (B-5) est connu sous le nom « Zannan 44-0082 (Strem) ».

Procédé de synthèse

Le procédé de synthèse selon l'invention (phase de production) est mis en oeuvre dans au moins un réacteur. La réaction de métathèse croisée est de préférence effectuée en milieu liquide, dans un solvant, notamment le toluène.

Le réacteur contient initialement une partie des réactifs, et la réaction est déclenchée par ajout de catalyseur. De préférence une partie supplémentaire des réactifs est introduite au cours du temps dans le réacteur. Le flux produit issu du réacteur peut être prélevé en continu ou par vidanges partielles espacées, mais de préférence il est prélevé seulement à la fin de la phase de production.

Les réactifs et produits de réactions peuvent être séparés selon des techniques connues de l'homme de l'art comme la distillation. Le catalyseur dégradé de métathèse peut être récupéré par adsorption, en particulier sur un adsorbant de type silice, alumine, charbon ou résine, ou par extraction liquide.

Les réactifs supplémentaires et le catalyseur introduits tout au long de la réaction peuvent être apportés à des instants déterminés, ou bien, de préférence, en continu. Avantageusement, le procédé est ainsi de type semi-continu, et avantageusement le taux d'alimentation de chaque espèce est constant au cours du temps ; mais les composés gazeux produits lors de la réaction (tels que l'éthylène) sont éliminés du réacteur en continu, assurant ainsi que les réactions de métathèse croisée et d'homométathèse ne sont pas équilibrées mais sont déplacées.

Ainsi, typiquement, on introduit une quantité initiale de premier composé insaturé et de deuxième composé insaturé dans le réacteur. Puis la réaction démarre lorsqu'on commence à introduire de manière continue du catalyseur ainsi qu'un flux de deuxième composé insaturé complémentaire. Les composés gazeux produits sont éliminés en continu. La réaction est stoppée au bout d'un certain temps par l'arrêt de l'alimentation du réacteur en catalyseur et en deuxième composé insaturé.

L'ajout progressif du catalyseur (de préférence en continu) permet de minimiser la consommation de celui-ci. En effet, le catalyseur se désactive très rapidement dans les conditions de la réaction. Cet ajout progressif permet aussi d'éviter l'apparition d'une concentration excessive de composé insaturé léger dans la solution (éthylène notamment), qui constitue un poison de la réaction.

L'invention prévoit l'utilisation de deux catalyseurs distincts, à savoir un premier catalyseur puis un deuxième catalyseur.

Selon un mode de réalisation préféré, on introduit le premier catalyseur uniquement dans le réacteur pendant une première durée $T_1$, puis on introduit le deuxième catalyseur uniquement dans le réacteur pendant une deuxième durée $T_2$. L'introduction du premier catalyseur est avantageusement continue pendant la durée $T_1$ (de préférence avec un taux d'alimentation constant) et l'introduction du deuxième catalyseur est avantageusement continue pendant la durée $T_2$ (de préférence avec un taux d'alimentation constant). Les taux d'alimentation du premier catalyseur et du deuxième catalyseur peuvent être différents.

Pour des raisons de mise en oeuvre pratique, il peut y avoir une période transitoire au cours de laquelle les deux catalyseurs sont introduits simultanément lors du changement de catalyseur.

Alternativement, on peut prévoir :

- l'injection d'un premier mélange des deux catalyseurs pendant la durée $T_1$ puis l'injection d'un deuxième mélange des deux catalyseurs pendant une durée $T_2$, le premier mélange étant plus riche en premier catalyseur que le deuxième mélange ; ou
- l'injection du premier catalyseur uniquement pendant la durée $T_1$ puis l'injection d'un mélange des deux catalyseurs pendant la durée $T_2$ ; ou
- l'injection du mélange des deux catalyseurs pendant la durée $T_1$ puis l'injection du deuxième catalyseur uniquement pendant la durée $T_2$ ; ou
- une modification continue de la composition du flux de catalyseur alimentant le réacteur, avec un enrichissement progressif en deuxième catalyseur et un appauvrissement progressif en premier catalyseur.

On peut également prévoir l'introduction de plus de deux catalyseurs, et par exemple :

- l'injection d'un premier catalyseur seul pendant la durée $T_1$ puis l'injection d'un deuxième catalyseur seul pendant la durée $T_2$, puis l'injection d'un troisième catalyseur seul pendant une durée supplémentaire $T_3$; ou
- l'injection d'un premier catalyseur seul pendant la durée $T_1$ puis l'injection d'un mélange de deux catalyseurs pendant la durée $T_2$ (dans ce cas, ce mélange de deux catalyseurs est considéré comme étant « *le deuxième catalyseur* » aux fins de la description ci-dessous) ; ou
- l'injection d'un mélange de deux catalyseurs pendant la durée $T_1$ puis l'injection d'un autre catalyseur pendant la durée $T_2$ (dans ce cas, le mélange de deux catalyseurs est considéré comme étant « *le premier catalyseur* » et l'autre catalyseur est « *le deuxième catalyseur* » aux fins de la description ci-dessous) ; et ainsi de suite.

Méthode générale de détermination des caractéristiques de l'alimentation en catalyseur

L'invention prévoit d'effectuer une analyse visant à déterminer les caractéristiques d'alimentation en catalyseurs permettant d'obtenir des performances souhaitées pour la réaction. La phase d'analyse en tant que telle peut être intégrée ou non au procédé de synthèse en tant qu'étape préliminaire.

L'analyse en question peut avoir pour objet :

- d'identifier, parmi deux catalyseurs donnés, l'ordre optimal d'utilisation des catalyseurs ;
- de choisir, parmi un ensemble de catalyseurs donnés, un premier catalyseur et un deuxième catalyseur, donnant des résultats optimaux ;
- de déterminer, pour une succession donnée d'un premier catalyseur et d'un deuxième catalyseur, la durée $T_1$ au bout de laquelle est effectué le passage du premier vers le deuxième catalyseur.

Le choix de l'ordre optimal de deux catalyseurs, celui d'un couple optimal de deux catalyseurs, et celui d'une durée d'injection optimale de catalyseur, dépendent des conditions dans lesquelles la synthèse est effectuée (température, pression, concentrations des espèces).

Par conséquent, ces différentes déterminations sont réalisées au moyen d'expériences effectuées dans des « *procédés de référence ».* Chaque procédé de référence est effectué dans les mêmes conditions de température, de pression, de stoechiométrie et d'alimentation en réactifs que celles de la synthèse qui est envisagée.

La composition du milieu réactionnel est analysée par prélèvement d'échantillons à intervalles de temps réguliers, dans chaque procédé de référence. Cela permet de déterminer à chaque instant d'une part le taux de conversion du premier composé insaturé (ou taux de transformation global, TTG), qui correspond à la fraction du premier composé insaturé ayant réagi, et d'autre part le rendement de la réaction (ou taux de transformation unitaire, TTU) en produit insaturé ($TTU_1$) et en coproduit insaturé ($TTU_2$), ce rendement correspondant au rapport du nombre de moles de réactif effectivement transformé en produit (ou en coproduit, et dans ce cas il y a 2 moles de réactif par mole de coproduit) sur le nombre de moles de réactif introduit dans le milieu réactionnel. Les TTG et TTU prennent des valeurs comprises entre 0 et 1, bornes comprises, c'est-à-dire entre 0 et 100 %.

Au cours du temps, le taux de conversion croît de 0 % à une valeur qui peut atteindre plus de 70 %, ou plus de 75 %, ou plus de 80 %, ou plus de 85 %, voire plus de 90 %. Ainsi, il est possible d'établir le rendement en produit insaturé en fonction du taux de conversion, et le rendement en coproduit insaturé en fonction du taux de conversion.

Pour un couple de catalyseurs donné, les inventeurs ont découvert que les performances du procédé de synthèse sont les meilleures lorsque le premier catalyseur est celui présentant le nombre de rotations ($TON_c$) le plus grand des deux, les TON des deux catalyseurs étant calculés dans les procédés de référence respectifs, et pour un taux de conversion $TTG_c$ cible donné.

Le TON est défini comme étant le nombre de moles de premier composé insaturé converties par mole de catalyseur. Il est calculé selon la formule suivante $TON=C_0 \times TTG/n$ où $C_0$ représente le nombre de moles de premier composé insaturé introduit dans le réacteur et n représente le nombre de moles de catalyseur introduits dans le réacteur.

La comparaison des catalyseurs respectifs est effectuée pour $TTG=TTG_c$, qui représente le taux de conversion cible.

$TTG_c$ peut valoir par exemple de 30 % à 95 %, de préférence de 40 % à 90 %, notamment de 50 à 80 %, plus particulièrement de 60 à 75 %.

Les inventeurs ont constaté que les catalyseurs ayant un TON élevé ont généralement une sélectivité relativement médiocre, et vice versa.

Par conséquent, dans le procédé de synthèse en tant que tel, l'utilisation du catalyseur avec le TON le plus élevé en premier permet de pousser la conversion du premier composé insaturé le plus loin possible, en minimisant la consommation de catalyseur. Puis, dans un deuxième temps, l'utilisation du catalyseur avec un TON plus faible permet de corriger les excès de la première partie du procédé, en convertissant le coproduit insaturé qui a été généré en quantité relativement importante lors de cette première partie, en le produit insaturé d'intérêt.

Les inventeurs ont constaté que, parmi les catalyseurs listés ci-dessus, ceux répondant à la formule générale (A) présentent un TON nettement plus élevé que ceux répondant à la formule générale (B). C'est pourquoi il est généralement avantageux d'utiliser un catalyseur de formule (A) en tant que premier catalyseur, puis un catalyseur de formule (B) en tant que deuxième catalyseur.

Par ailleurs, les inventeurs ont également constaté que, parmi les catalyseurs listés ci-dessus, ceux des formules (A-1) à (A-10) présentent un TON nettement plus élevé que ceux des formules (B-1) à (B-5). C'est pourquoi il est généralement avantageux d'utiliser un catalyseur de formule (A-1) à (A-10) en tant que premier catalyseur, puis un catalyseur de formule (B-1) à (B-5) en tant que deuxième catalyseur.

S'agissant maintenant du choix d'un couple optimal de catalyseurs parmi un groupe de catalyseurs possibles, on choisit avantageusement :

- en tant que premier catalyseur, le catalyseur du groupe ayant le TON maximal dans le procédé de référence (pour le taux de conversion cible $TTG_c$); et
- en tant que deuxième catalyseur :

  o soit celui qui, parmi les catalyseurs restants du groupe, présente un facteur de pente p minimal (et lorsque plusieurs catalyseurs ont des facteurs de pente p égaux ou très proches, on choisit celui ayant le TON maximal) ;
  o soit celui qui, parmi les catalyseurs restants du groupe, présente un paramètre $\beta$, ou selon une variante un paramètre $\beta'$, qui est maximal (et lorsque plusieurs catalyseurs ont des paramètres $\beta'$ égaux ou très proches, on choisit le catalyseur pour lequel un paramètre $\gamma'$ est minimal).

Les deux critères possibles pour le choix du deuxième catalyseur visent à assurer que ce deuxième catalyseur est celui permettant la meilleure conversion possible du coproduit insaturé.
Le calcul de p, $\beta$, $\beta'$ et $\gamma'$ en fonction des résultats du procédé de référence est détaillé ci-dessous en relation avec un exemple de réaction.
En ce qui concerne enfin la durée $T_1$ au bout de laquelle est effectué le passage du premier vers le deuxième catalyseur, elle est choisie comme étant égale, ou comme étant supérieure à l'un de deux seuils $T_{S1}$ et $T_{S2}$. Elle peut par exemple être choisie comme étant supérieure ou égale au minimum de ces deux seuils, ou idéalement supérieure ou égale au maximum de ces deux seuils.
Le seuil $T_{S1}$ correspond à la durée au bout de laquelle, dans le procédé de référence, le taux de conversion du premier composé insaturé $TTG_S$ et le rendement en coproduit insaturé $TTU_{2S}$ sont reliés par l'équation $TTU_{2S}=TTU^*\times(TTG_S-1)/(TTG^*-1)$, où TTU* représente un rendement en coproduit insaturé souhaité à l'issue de la phase de production, et TTG* représente un taux de conversion du premier composé insaturé souhaité à l'issue de la phase de production.
Le seuil $T_{S2}$ correspond à la durée au bout de laquelle, dans le procédé de référence, la dérivée seconde de TON(t) (nombre de rotations du premier catalyseur en fonction du temps) s'annule.
En général, la durée $T_1$ au bout de laquelle est effectué le passage du premier vers le deuxième catalyseur est telle que le taux de conversion du premier composé insaturé vaut, à la fin de la durée $T_1$, de 30 à 90 %, de préférence de 40 à 80 %, et plus particulièrement de 50 à 70 %.

Méthode de calcul des paramètres TON, p, $\beta$, $\beta'$, $\gamma'$, $T_{S1}$ et $T_{S2}$.

Ce qui précède pourra être compris plus facilement en faisant référence à l'exemple dans lequel le premier composé insaturé est le 9-décénoate de méthyle (ou DM), le deuxième composé insaturé est l'acrylonitrile (ACN), le produit insaturé est le 10-cyano-9-décénoate de méthyle (NE) et le coproduit insaturé est le 9-octadécénedioate de méthyle (DE).
L'ACN est un composé léger, qui a un point d'ébullition inférieur à 100°C alors que le DM a un point d'ébullition supérieur à 200°C.
La réaction de métathèse croisée souhaitée est la réaction : ACN + DM $\rightarrow$ NE + éthylène. L'éthylène produit est rapidement éliminé en phase gaz par entraînement avec le solvant qui est à son point d'ébullition. Le solvant est condensé et retourné au réacteur. Du fait de l'élimination continue de l'éthylène, la réaction n'est pas considérée comme équilibrée.
La réaction d'homométathèse est la réaction : DM + DM $\rightarrow$ DE + éthylène. Elle est également non équilibrée pour les mêmes raisons.
Une autre réaction qui a lieu est celle entre l'ACN et le produit d'homométathèse : ACN + DE $\leftrightarrow$ DM + NE, cette réaction redonnant le réactif initial (DM) et le produit recherché (NE). Cette réaction est équilibrée, le produit de la réaction souhaité réagissant avec le réactif initial pour donner le produit d'homométathèse. Cette réaction inverse est surtout présente à conversion élevée, lorsque la concentration en NE est élevée, et que la conversion du DM est déjà bien avancée.
Dans les procédés de référence, tout comme dans les procédés de synthèse en tant que tels, on introduit les catalyseurs successifs en continu, et on introduit l'ACN pour partie avant le début de la réaction et pour partie au cours de la réaction. L'ajout progressif de l'ACN est rendu nécessaire par la forte inhibition des catalyseurs par celui-ci. On ne peut donc avoir une forte teneur en ACN dès le début de la réaction.
Pour chaque catalyseur envisagé, on réalise un procédé de référence, qui est défini par une température et une pression de fonctionnement, ainsi que par un nombre de moles initial de DM dans le réacteur $C_0$ et un nombre de moles initial d'ACN dans le réacteur $C'_0$, et enfin par un taux d'alimentation du réacteur en ACN et un taux d'alimentation du réacteur en catalyseur.
Les mesures de composition du milieu réactionnel au cours du temps lors du procédé de référence permettent de déterminer les rendements en produit insaturé d'intérêt $TTU_1$ et en coproduit insaturé $TTU_2$ en fonction du taux de

conversion TTG, comme illustré sur la **figure 1.**

A partir de ces mesures, on peut déterminer :

- le paramètre a, qui est la valeur de la dérivée de la courbe $TTU_1$ pour TTG=0 (pente à l'origine de la courbe $TTU_1$ sur la figure) ;
- le paramètre b, qui est la valeur de la dérivée de la courbe $TTU_2$ pour TTG=0 (pente à l'origine de la courbe $TTU_2$ sur la figure) ;
- le facteur de pente p (mentionné ci-dessus), qui correspond à la valeur absolue (positive) de la dérivée de la courbe $TTU_2$ pour TTG=1 (pente de la courbe $TTU_2$ au point TTG=1 sur la figure).

Concernant ce dernier paramètre, les données expérimentales recueillies dans le procédé de référence ne vont en général pas jusqu'à un taux de conversion de 1 (ou 100 %), et il est donc nécessaire d'extrapoler à partir de l'allure de la courbe pour des taux de conversion inférieurs.

Les mesures de pentes fournissant les paramètres a, b et p sont illustrées sur la **figure 1.**

Le nombre de rotations du catalyseur, $TON_c$, est déterminé selon la formule $TON_c=C_0\times TTG_c/n$, où n représente le nombre de moles de catalyseurs introduites pour effectuer la réaction jusqu'au taux de conversion $TTG_c$.

On peut ensuite calculer un paramètre $\alpha$ selon la formule $\alpha=(2a/b)x(C_0/C'_0)$.

Pour chaque point expérimental, on peut calculer la fonction X selon la formule suivante : $X=((TTU_2/2)\times(z/C_0-TTU_1))/((1-TTG)\times TTU_1)$, z représentant le nombre total de moles d'ACN introduites dans le milieu réactionnel au point considéré (y inclus la quantité initiale $C'_0$).

Pour chaque point expérimental, on peut également calculer la fonction Y selon la formule suivante : $Y=((p\times\alpha)/(2+p))\times(z/C_0-TTU_1)/TTU_1 + ((2+2p)/(2+p))\times((1-TTG)/TTU_1)$.

Ensuite, on approxime (on ajuste) la fonction Y(X) avec un modèle linéaire ou affine. Le modèle linéaire est de la forme $Y=\beta\times X$ tandis que le modèle affine est de la forme $Y=\beta'\times X-\gamma'$, pour les données expérimentales obtenues pour les conversions les plus élevées, c'est-à-dire celles obtenues au-delà de la conversion correspondant au maximum de rendement en coproduit insaturé. Il est préférable d'utiliser le modèle affine, si les données expérimentales le permettent.

L'ajustement ou approximation peut être effectué par exemple en utilisant la méthode des moindres carrés.

En fonction de l'ensemble de ces résultats, il est possible de comparer différents catalyseurs selon les valeurs du TON et des paramètres p, $\beta$, $\beta'$ et $\gamma'$, comme exposé ci-dessus.

Concernant le calcul de la durée seuil $T_{S1}$, il est fait référence à la **figure 2,** sur laquelle est représenté le rendement en coproduit $TTU_2$ en fonction du taux de conversion TTG.

On détermine un taux de conversion TTG* et un rendement en coproduit TTU* qui sont souhaités à l'issue de la phase de production, et on trace sur le graphique la droite reliant ce point au point [TTG=1 ;$TTU_2$=0]. On détermine le point d'intersection [$TTG_s$ ; $TTU_{2S}$] entre cette droite et la courbe expérimentale $TTU_2$(TTG) pour le premier catalyseur dans ses conditions de référence. La durée seuil $T_{S1}$ correspond au temps nécessaire pour atteindre le taux de conversion en ce point.

En ce qui concerne la détermination de la durée seuil $T_{S2}$, on peut tracer la courbe du TON en fonction du temps et mesurer le temps pour lequel cette courbe présente un point d'inflexion (dérivée seconde s'annule).

Le travail qui a conduit à cette invention a reçu un financement de la part de l'Union Européenne dans le cadre du 7ème Programme Cadre (FP7/2007-2013) sous le numéro de projet N°241718 EUROBIOREF.

EXEMPLES

Les exemples suivants illustrent l'invention sans la limiter.

Exemple 1 - métathèse croisée 9-décénoate de méthyle / acrylonitrile, procédé de référence

On met en oeuvre la réaction suivante :

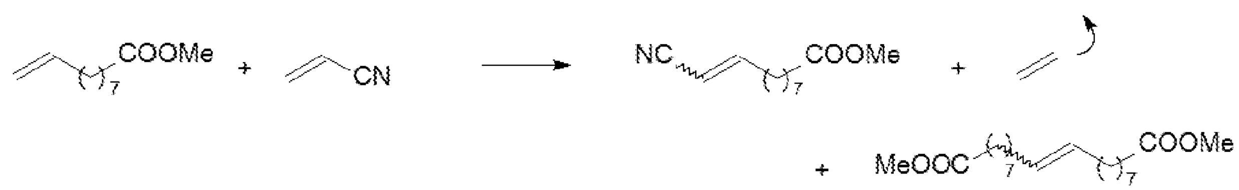

en utilisant le catalyseur M71-SiPr fourni par la société Umicore.

Dans un réacteur en verre de 250 ml muni d'un réfrigérant et purgé à l'azote, on charge 15 g de 9-décénoate de méthyle (81,4 mmol) préparé conformément à l'exemple 1 du document US 2011/0113679, préalablement passé sur colonne d'alumine, 2,15 g d'acrylonitrile (40,7 mmol) et 150 g de toluène séché sur tamis moléculaire. On chauffe à 110°C et on ajoute via des seringues montées sur pousse-seringues, sur une période de 2 heures, 2,6 g d'acrylonitrile (49 mmol) et 2 mg de catalyseur M71-SiPr ($2,44\times10^{-6}$ mol) dissous dans 5 g de toluène. Des prélèvements sont réalisés toutes les 30 minutes pour analyse par chromatographie en phase gaz. Les conversions du 9-décénoate de méthyle (DM) et les rendements en nitrile-ester insaturé en $C_{11}$ (NE) et en diester insaturé en $C_{18}$ (DE) sont reportées sur le graphe de la **figure 3.**

On constate que, pour obtenir un taux de de conversion de 80 %, le TON est de 35800.

A partir des résultats expérimentaux, on détermine les valeurs suivantes pour les paramètres d'intérêt :

- p=1,31 ;
- a=0,44 ;
- b=0,56 ;
- $\alpha$=3,18.

Par ailleurs, l'ajustement linéaire de la fonction Y(X) fournit la valeur de 2,72 pour le paramètre $\beta$.

Exemple 2 - métathèse croisée 9-décénoate de méthyle / acrylonitrile, procédé de référence

On répète le procédé de référence pour la métathèse croisée entre le 9-décénoate de méthyle et l'acrylonitrile avec un autre catalyseur, à savoir le catalyseur Hoveyda-Grubbs 2 (fourni par la société Aldrich).

Dans un réacteur en verre de 250 ml muni d'un réfrigérant et purgé à l'azote, on charge 15 g de 9-décénoate de méthyle (81,4 mmol) préalablement passé sur colonne d'alumine, 2,15 g d'acrylonitrile (40,7 mmol) et 150 g de toluène séché sur tamis moléculaire. On chauffe à 110°C et on ajoute via des seringues montées sur pousse-seringues, sur une période de 2 heures, 2,6 g d'acrylonitrile (49 mmol) et 5,1 mg de catalyseur Hoveyda-Grubbs 2 ($8,1\times10^{-6}$ mol) dissous dans 5 g de toluène. Des prélèvements sont réalisés toutes les 15 minutes pour analyse par chromatographie en phase gaz. Les conversions du 9-décénoate de méthyle (DM) et les rendements en nitrile-ester insaturé en $C_{11}$ (NE) et du diester insaturé en $C_{18}$ (DE) sont reportées sur le graphe de la **figure 4.**

On constate que, pour obtenir un taux de de conversion de 80 %, le TON est de 12600.

A partir des résultats expérimentaux, on détermine les valeurs suivantes pour les paramètres d'intérêt :

- p=0,63 ;
- a=0,78 ;
- b=0,24 ;
- $\alpha$=12,89.

Par ailleurs, l'ajustement linéaire de la fonction Y(X) fournit la valeur de 16,2 pour le paramètre $\beta$.

Exemple 3 - métathèse croisée 9-décénoate de méthyle / acrylonitrile, procédé selon l'invention

On met en oeuvre la réaction de métathèse croisée en utilisant successivement le catalyseur M71-SiPr puis le catalyseur Hoveyda-Grubbs 2, et ce dans les mêmes conditions que les exemples 1 et 2.

Dans un réacteur en verre de 250 ml muni d'un réfrigérant et purgé à l'azote, on charge 15 g de 9-décénoate de méthyle (81,4 mmol) préalablement passé sur colonne d'alumine, 2,15 g d'acrylonitrile (40,7 mmol) et 150 g de toluène séché sur tamis moléculaire. On chauffe à 110°C et on ajoute via des seringues montées sur pousse-seringues, sur une période de 1 heure, 1,3 g d'acrylonitrile (25 mmol) et 1 mg de catalyseur M71-SiPr ($1,22\times10^{-6}$ mol) dissous dans 5 g de toluène.

On continue ensuite la réaction en ajoutant, sur une période de 1 heure, 1,3 g d'acrylonitrile (25 mmol) et 1,5 mg de catalyseur Hoveyda-Grubbs 2 ($2,45\times10^{-6}$ mol) dissous dans 5 g de toluène.

Les conversions du 9-décénoate de méthyle (DM) et les rendements en nitrile-ester insaturé en $C_{11}$ (NE) et du diester insaturé en $C_{18}$ (DE) sont reportés sur le graphe de la **figure 5.**

Par rapport au catalyseur M71-SiPr seul, cet exemple montre qu'il est possible de réduire le rendement final en diester (12% contre 18% dans l'essai de référence).

Par rapport au catalyseur Hoveyda-Grubbs 2 seul, cet essai montre qu'il est possible d'utiliser moins de catalyseur (45 ppm molaire contre 100 ppm) dans l'essai de référence pour obtenir des rendements voisins en diester.

Exemple 4 - métathèse croisée 9-décénoate de méthyle / acrylonitrile (comparatif)

On met en oeuvre la réaction de métathèse croisée en utilisant successivement le catalyseur Hoveyda-Grubbs 2 puis le catalyseur M71-SiPr, et ce dans les mêmes conditions que les exemples 1 et 2.

Dans un réacteur en verre de 250 ml muni d'un réfrigérant et purgé à l'azote, on charge 15 g de 9-décénoate de méthyle (81,4 mmol) préalablement passé sur colonne d'alumine, 2,15 g d'acrylonitrile (40,7 mmol) et 150 g de toluène séché sur tamis moléculaire. On chauffe à 110°C et on ajoute via des seringues montées sur pousse-seringues, sur une période de 1 heure, 1,3 g d'acrylonitrile (25 mmol) et 1,5 mg de catalyseur Hoveyda-Grubbs 2 ($2,45\times10^{-6}$ mol) dissous dans 5 g de toluène.

On continue ensuite la réaction en ajoutant, sur une période de 1 heure, 1,3 g d'acrylonitrile (25 mmol) et 1 mg de catalyseur M71-SiPr ($1,22\times10^{-6}$ mol) dissous dans 5 g de toluène.

Les conversions du 9-décénoate de méthyle (DM) et les rendements en nitrile-ester insaturé en $C_{11}$ (NE) et du diester insaturé en $C_{18}$ (DE) sont reportés sur le graphe de la **figure 6.**

Exemple 5 - métathèse croisée 9-décénoate de méthyle / acrylonitrile (comparatif)

On met en oeuvre la réaction de métathèse croisée en utilisant simultanément les catalyseurs M71-SiPr et Hoveyda-Grubbs 2, et ce dans les mêmes conditions que les exemples 1 et 2.

Dans un réacteur en verre de 250 ml muni d'un réfrigérant et purgé à l'azote, on charge 15 g de 9-décénoate de méthyle (81,4 mmol) préalablement passé sur colonne d'alumine, 2,15 g d'acrylonitrile (40,7 mmol) et 150 g de toluène séché sur tamis moléculaire. On chauffe à 110°C et on ajoute via des seringues montées sur pousse-seringues, sur une période de 2 heure, 2,6 g d'acrylonitrile (25 mmol), 1 mg de catalyseur M71-SiPr ($1,22\times10^{-6}$ mol) et 1,5 mg de catalyseur Hoveyda-Grubbs 2 ($2,45\times10^{-6}$ mol) dissous dans 5 g de toluène.

Les conversions du 9-décénoate de méthyle (DM) et les rendements en nitrile-ester insaturé en $C_{11}$ (NE) et du diester insaturé en $C_{18}$ (DE) sont reportés sur le graphe de la **figure 7.**

## Revendications

1. Procédé de synthèse d'un produit insaturé par métathèse croisée entre un premier composé insaturé comportant au moins 8 atomes de carbone et un deuxième composé insaturé comportant moins de 8 atomes de carbone, le premier composé insaturé étant susceptible de produire un coproduit insaturé, comportant plus de 14 atomes de carbone, par homométathèse, ledit procédé comprenant au moins une phase de production qui comprend :

   - l'alimentation d'un réacteur avec le premier composé insaturé ;
   - l'alimentation du réacteur avec le deuxième composé insaturé ;
   - l'alimentation du réacteur avec au moins un premier catalyseur de métathèse suivie par l'alimentation du réacteur avec au moins un deuxième catalyseur de métathèse ;
   - le prélèvement d'un flux produit issu du réacteur ;

   le nombre de rotations du premier catalyseur étant supérieur au nombre de rotations du deuxième catalyseur pour atteindre un même taux de conversion cible du premier composé insaturé, les nombres de rotations des catalyseurs étant déterminés dans des conditions de température, de pression, de stoechiométrie, et de concentrations de réactifs identiques à celles de la phase de production.

2. Procédé selon la revendication 1, dans lequel le taux de conversion cible vaut de 30 % à 95 %, de préférence de 40 % à 90 %, notamment de 50 à 80 %, plus particulièrement de 60 à 75 %.

3. Procédé selon la revendication 1 ou 2, dans lequel la phase de production est semi-continue, et comprend de préférence successivement :

   (1) l'alimentation du réacteur avec la totalité du premier composé insaturé et une première partie du deuxième composé insaturé, avant le démarrage de la réaction ;
   (2) l'alimentation progressive du réacteur avec une deuxième partie du deuxième composé insaturé et avec le premier catalyseur ;
   (3) l'alimentation progressive du réacteur avec une troisième partie du deuxième composé insaturé et avec le deuxième catalyseur.

**4.** Procédé selon l'une des revendications 1 à 3, dans lequel :

- le premier composé insaturé est de formule :

(I) $R_1\text{-CH=CH-}(CH_2)_n\text{-}R_2$ ;

- le deuxième composé insaturé est de formule :

(II) $R_3\text{-CH=CH-}R_4$ ;

- le produit insaturé est de formule :

(III) $R_4\text{-CH=CH-}(CH_2)_n\text{-}R_2$ ;

- le coproduit insaturé est de formule :

(IV) $R_2\text{-}(CH_2)_n\text{-CH=CH-}(CH_2)_n\text{-}R_2$ ;

$R_1$ représentant un atome d'hydrogène ou un radical alkyle ou alkényle comportant de 1 à 8 atomes de carbone ; $R_2$ représentant $COOR_5$ ou CN ou CHO ou $CH_2OH$ ou $CH_2Cl$ ou $CH_2Br$ ; $R_3$ et $R_4$ représentant chacun un atome d'hydrogène ou un radical alkyle comportant de 1 à 4 atomes de carbone ou $COOR_5$ ou CN ou CHO ou $CH_2OH$ ou $CH_2Cl$ ou $CH_2Br$, $R_3$ et $R_4$ étant identiques ou différents et ne comportant au total pas plus de 6 atomes de carbone ; $R_5$ représentant un atome d'hydrogène ou un radical alkyle comportant de 1 à 4 atomes de carbone ; et n étant un nombre entier de 4 à 11.

**5.** Procédé selon l'une des revendications l'une des revendications 1 à 4, dans lequel le deuxième composé insaturé est un acrylate ou de préférence l'acrylonitrile, le premier composé insaturé est un acide, un nitrile insaturé ou un ester insaturé, de préférence choisi parmi le 9-décénoate de méthyle, le 9-décènenitrile, le 10-undécènenitrile et le 10-undécénoate de méthyle, le produit insaturé est un nitrile-ester, un nitrile-acide, un dinitrile ou un diester insaturé, et le coproduit insaturé est un diester, un dinitrile ou un diacide insaturé.

**6.** Procédé selon l'une des revendications 1 à 5, dans lequel les réactions de métathèse sont effectuées en phase liquide, le cas échéant dans un solvant, et résultent de préférence en la production d'au moins un composé insaturé sous forme gazeuse, de manière plus particulièrement préférée l'éthylène, dans le réacteur, le procédé comprenant le soutirage de celui-ci du réacteur de manière continue.

**7.** Procédé selon l'une des revendications 1 à 6, dans lequel le taux de conversion du premier composé insaturé à l'issue de la phase de production est de 50 à 98 %, de préférence de 60 à 95 %, de manière plus particulièrement préférée de 70 à 90 %.

**8.** Procédé selon l'une des revendications 1 à 7, comprenant une phase préliminaire de sélection du premier catalyseur et du deuxième catalyseur parmi un ensemble de catalyseurs possibles, avant la phase de production.

**9.** Procédé selon la revendication 8, dans lequel la phase préliminaire de sélection comprend :

- la mise en oeuvre d'un procédé de référence pour chaque catalyseur possible, chaque procédé de référence comprenant l'alimentation du réacteur avec le premier composé insaturé, l'alimentation du réacteur avec le deuxième composé insaturé et l'alimentation du réacteur avec le catalyseur, les conditions de température, de pression, de stoechiométrie et de concentrations de réactifs pour chaque procédé de référence étant identiques à celles utilisées pour la phase de production ;
- la collecte des résultats des procédés de référence, comprenant la mesure de concentrations d'espèces chimiques dans le réacteur au cours du temps ;
- le choix du premier catalyseur et du deuxième catalyseur en fonction des résultats des procédés de référence.

**10.** Procédé selon la revendication 9, dans lequel le premier catalyseur est le catalyseur présentant le nombre de rotations maximal dans le procédé de référence, pour un même taux de conversion cible du premier composé insaturé.

**11.** Procédé selon la revendication 9 ou 10, dans lequel le deuxième catalyseur est un catalyseur pour lequel, dans le procédé de référence, la valeur absolue de la dérivée du rendement de la réaction en coproduit insaturé en fonction du taux de conversion du premier composé insaturé, déterminée au taux de conversion de 1, est minimale.

**12.** Procédé selon la revendication 11, dans lequel le deuxième catalyseur est choisi, parmi les catalyseurs pour lesquels, dans le procédé de référence, la valeur absolue de la dérivée du rendement de la réaction en coproduit insaturé en fonction du taux de conversion du premier composé insaturé mesurée au taux de conversion de 1 est minimale, comme étant le catalyseur pour lequel le nombre de rotations dans le procédé de référence est maximal, pour un même taux de conversion cible du premier composé insaturé.

**13.** Procédé selon la revendication 9 ou 10, dans lequel :

- on calcule à différents instants de chaque procédé de référence le nombre X qui est défini par la formule suivante :

$$(1) \qquad X=(TTU_2/2)\times(z/C_0-TTU_1)/((1-TTG)\times TTU_1),$$

dans laquelle $C_0$ représente le nombre de moles du premier composé insaturé dans le réacteur avant le début de l'alimentation en catalyseur, TTG représente le taux de conversion du premier composé insaturé à l'instant considéré, $TTU_1$ représente le rendement de la réaction en produit insaturé à l'instant considéré, $TTU_2$ représente le rendement de la réaction en coproduit insaturé à l'instant considéré, et z représente le nombre total cumulé de moles de deuxième composé insaturé introduites dans le réacteur à l'instant considéré ;
- on calcule à différents instants de chaque procédé de référence le nombre Y qui est défini par la formule suivante :

$$(2) \qquad Y=(p\times\alpha/(2+p))\times(z/(C_0\times TTU_1)-1) + (2+2p)\times(1-TTG)/((2+p)\times TTU_1)$$

dans laquelle $C_0$, TTG, $TTU_1$ et z ont la signification ci-dessus, p représente la valeur absolue de la dérivée de la fonction $TTU_2$(TTG) pour un taux de conversion égal à 1, et $\alpha$ vaut $(2a/b)\times(C_0/C'_0)$, a représentant la dérivée de la fonction $TTU_1$(TTG) pour un taux de conversion égal à 0, b représentant la dérivée de la fonction $TTU_2$(TTG) pour un taux de conversion égal à 0 et $C'_0$ représentant le nombre de moles du deuxième composé insaturé dans le réacteur avant le début de l'alimentation en catalyseur ;
- on approxime la fonction Y(X) par une équation $Y=\beta\times X$ ou, de manière préférée, par une équation $Y=\beta'\times X-\gamma'$ ;
- on sélectionne le deuxième catalyseur comme étant le catalyseur pour lequel le paramètre $\beta$ ou $\beta'$ est maximal.

**14.** Procédé selon l'une des revendications 1 à 13, dans lequel le réacteur est alimenté en premier catalyseur jusqu'à ce que le taux de conversion du premier composé insaturé atteigne une valeur seuil, ladite valeur seuil étant de 30 à 90 %, de préférence de 40 à 80 %, et plus particulièrement de 50 à 70 %.

**15.** Procédé selon l'une des revendications 1 à 14, dans lequel le réacteur est alimenté en premier catalyseur pendant une durée égale, ou de préférence supérieure, à une durée seuil, ladite durée seuil étant celle au bout de laquelle le taux de conversion du premier composé insaturé $TTG_S$ et le rendement en coproduit insaturé $TTU_{2S}$, dans un procédé de référence, sont reliés par l'équation $TTU_{2S}=TTU^*\times(TTG_S-1)/(TTG^*-1)$, où $TTU^*$ est la valeur du rendement en coproduit insaturé souhaité à l'issue de la phase de production, et $TTG^*$ est la valeur du taux de conversion du premier composé insaturé souhaité à l'issue de la phase de production, le procédé de référence comprenant l'alimentation du réacteur avec le premier composé insaturé, l'alimentation du réacteur avec le deuxième composé insaturé et l'alimentation du réacteur avec le premier catalyseur, les conditions de température, de pression, de stoechiométrie et de concentrations de réactifs pour le procédé de référence étant identiques à celles utilisées pendant la phase de production.

**16.** Procédé selon l'une des revendications 1 à 15, dans lequel le réacteur est alimenté en premier catalyseur pendant une durée égale, ou de préférence supérieure, à une durée seuil qui est la durée au bout de laquelle, dans un procédé de référence, la dérivée seconde du nombre de rotations du premier catalyseur en fonction du temps s'annule, le procédé de référence comprenant l'alimentation du réacteur avec le premier composé insaturé, l'alimentation du réacteur avec le deuxième composé insaturé et l'alimentation du réacteur avec le premier catalyseur, les conditions de température, de pression, de stoechiométrie et de concentrations de réactifs pour le procédé de référence étant identiques à celles utilisées pendant la phase de production.

**17.** Procédé selon l'une des revendications 1 à 16 comprenant, avant la phase de production, une phase préliminaire de détermination de la durée d'introduction du premier catalyseur, qui comprend :

- la mise en oeuvre du procédé de référence ;
- la collecte des résultats du procédé de référence, comprenant la mesure de concentrations d'espèces chimiques dans le réacteur au cours du temps ; et
- le calcul de la durée d'introduction du premier catalyseur en fonction des résultats des procédés de référence.

**18.** Procédé selon l'une des revendications 1 à 17, dans lequel le premier catalyseur et / ou le deuxième catalyseur sont des catalyseurs ruthénium-carbène, et de préférence sont choisis parmi les catalyseurs de formule (A) et (B) suivantes :

(A)

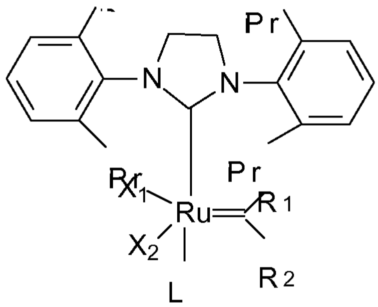

(B)

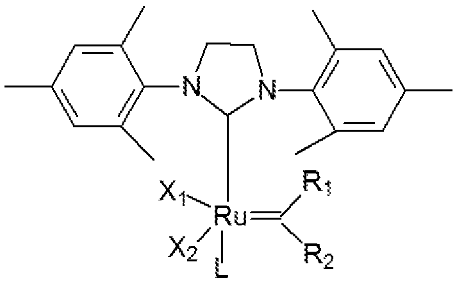

dans lesquelles $X_1$ et $X_2$ sont des ligands anioniques, L est un ligand neutre électrodonneur, et $R_1$ et $R_2$ représentent H ou un substituant contenant de 1 à 20 atomes de carbone de type alkyle, alkenyle, alkynyle, aryle, alcoxy, alkenyloxy, alkynyloxy, aryloxy, alcoxycarbonyle, alkylthiol, arylthiol, alkylsulfonyle, alkylsulfinyle ; le substituant contenant éventuellement des groupements de type hydroxyle, thiol, thioéther, cétone, aldéhyde, ester, éther, amine, imine, amide, nitro, acide carboxylique, disulfure carbonate, isocyanate, carbodiimide, carboalcoxy, carbamate ou halogène, $R_2$ étant de préférence lié à L pour former un ligand chélatant.

**19.** Procédé selon l'une des revendications 1 à 18, dans lequel le premier catalyseur et / ou le deuxième catalyseur sont choisis parmi les catalyseurs de formules (A-1) à (A-10) et (B-1) à (B-5) suivantes :

(A-1)

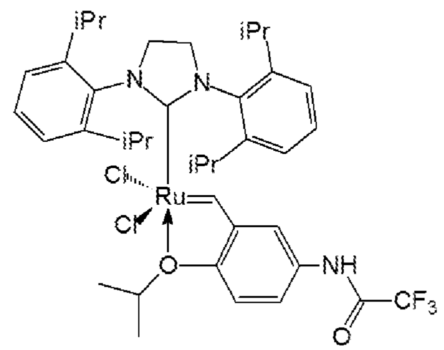

(A-2)

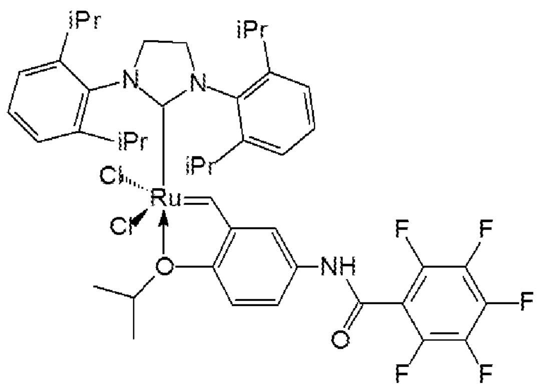

(A-3)

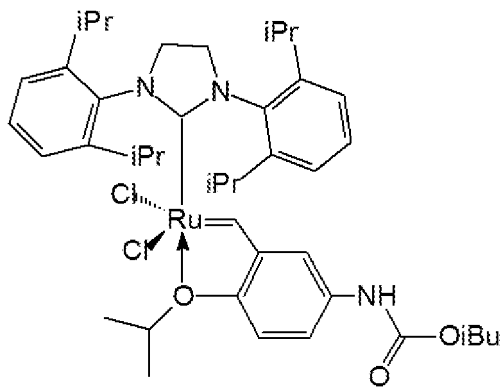

(A-4)

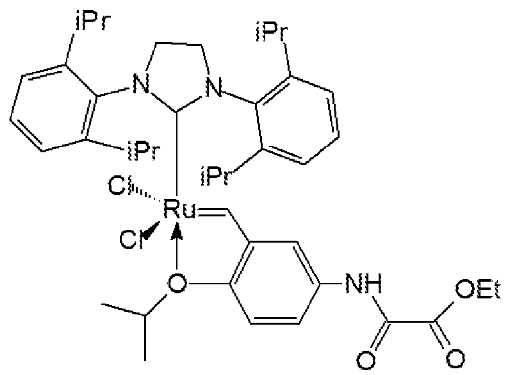

(A-5)

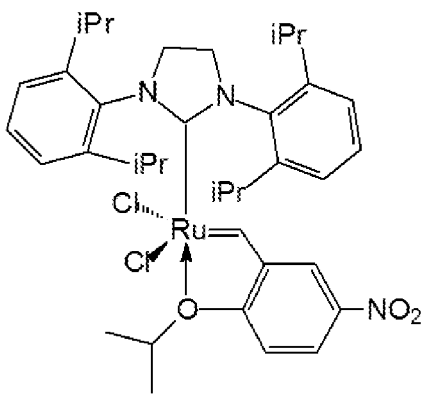

(A-6)

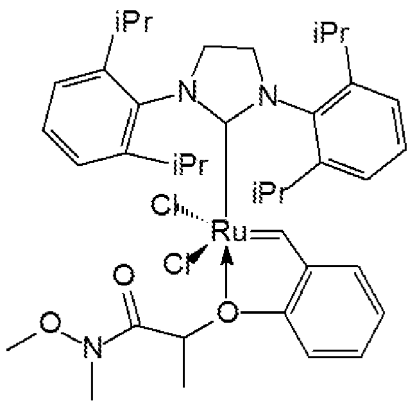

(A-7)

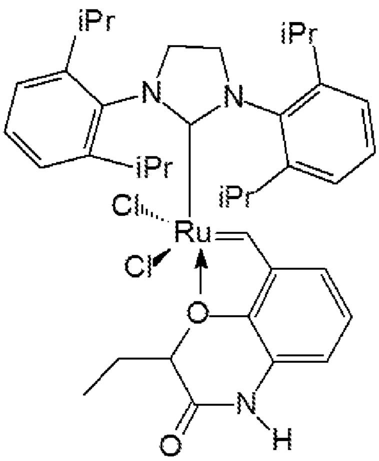

(A-8)

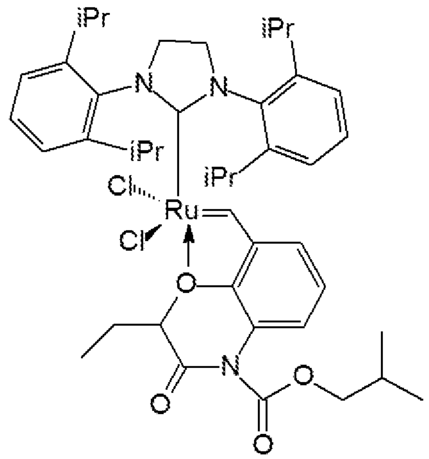

(A-9)

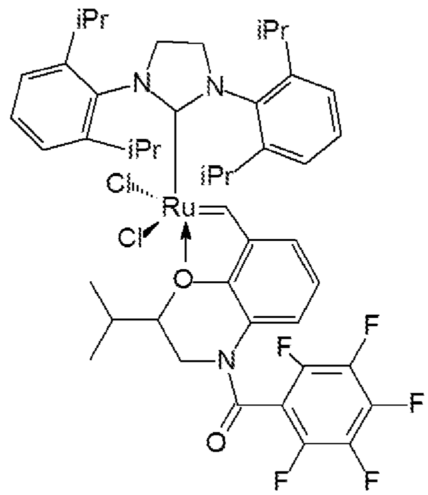

(A-10)

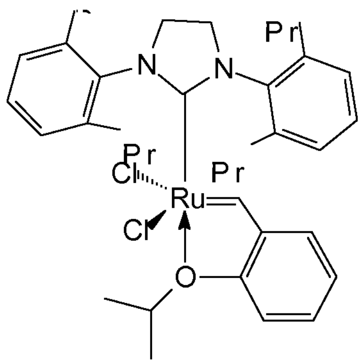

(B-1)

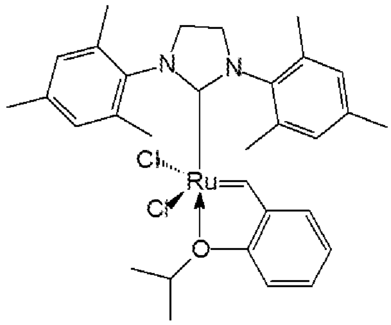

(B-2)

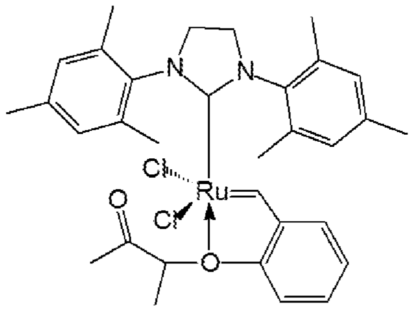

(B-3)

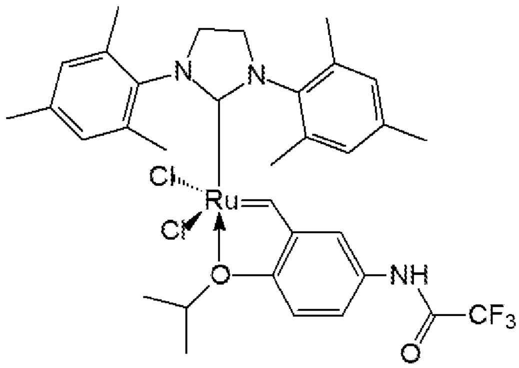

(B-4)

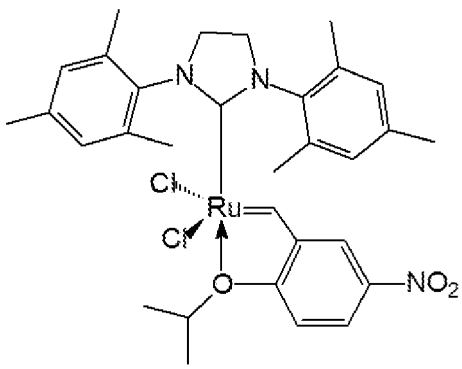

(B-5)

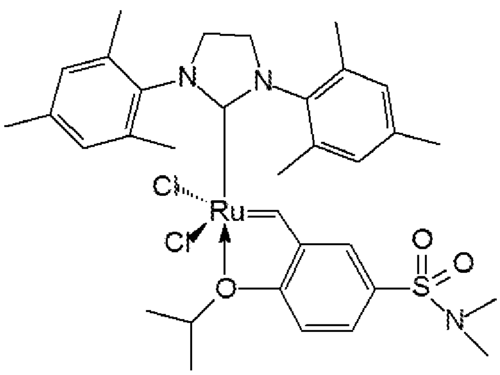

**20.** Procédé selon la revendication 18 ou 19, dans lequel le premier catalyseur est un catalyseur de formule (A) et dans lequel le deuxième catalyseur est un catalyseur de formule (B), ou de préférence dans lequel le premier catalyseur est choisi parmi les catalyseurs de formules (A-1) à (A-10) et le deuxième catalyseur est choisi parmi les catalyseurs de formules (B-1) à (B-5).

**21.** Procédé de synthèse d'un acide ou d'un ester $\alpha,\omega$-aminoalcanoïque, comprenant la synthèse d'un produit insaturé selon le procédé de l'une des revendications 1 à 20, qui est un nitrile-ester ou un nitrile-acide insaturé, et une réaction d'hydrogénation de celui-ci.

**Patentansprüche**

1. Verfahren zur Synthese eines ungesättigten Produkts durch Kreuzmetathese zwischen einer ersten ungesättigten Verbindung mit mindestens 8 Kohlenstoffatomen und einer zweiten ungesättigten Verbindung mit weniger als 8 Kohlenstoffatomen, wobei die erste ungesättigte Verbindung durch Homometathese ein ungesättigtes Coprodukt mit mehr als 14 Kohlenstoffatomen liefern kann, wobei das Verfahren mindestens eine Produktionsphase umfasst, die Folgendes umfasst:

   - die Beschickung eines Reaktors mit der ersten ungesättigten Verbindung;
   - die Beschickung des Reaktors mit der zweiten ungesättigten Verbindung;
   - die Beschickung des Reaktors mit mindestens einem ersten Metathesekatalysator, gefolgt von der Beschickung des Reaktors mit mindestens einem zweiten Metathesekatalysator;
   - Entnehmen eines erhaltenen Produktstroms aus dem Reaktor;

   wobei die Wechselzahl des ersten Katalysators größer ist als die Wechselzahl des zweiten Katalysators, damit der gleiche Ziel-Umwandlungsgrad des ersten ungesättigten Verbindung erhalten wird, wobei die Wechselzahlen der Katalysatoren unter identischen Bedingungen von Temperatur, Druck, Stöchiometrie und Konzentrationen der Reagenzien zu denjenigen der Produktionsphase bestimmt werden.

2. Verfahren nach Anspruch 1, wobei der Ziel-Umwandlungsgrad 30% bis 95%, vorzugsweise 40% bis 90%, insbesondere 50 bis 80%, ganz besonders 60 bis 75% beträgt.

3. Verfahren nach Anspruch 1 oder 2, wobei die Produktionsphase halbkontinuierlich ist und vorzugsweise nacheinander Folgendes umfasst:

   (1) Beschickung des Reaktors mit der Gesamtheit der ersten ungesättigten Verbindung und einem ersten Teil der zweiten ungesättigten Verbindung vor dem Beginn der Reaktion;
   (2) allmähliche Beschickung des Reaktors mit einem zweiten Teil der zweiten ungesättigten Verbindung und mit dem ersten Katalysator;
   (3) allmähliche Beschickung des Reaktors mit einem dritten Teil der zweiten ungesättigten Verbindung und mit dem zweiten Katalysator.

4. Verfahren nach einem der Ansprüche 1 bis 3, wobei:

   - die erste ungesättigte Verbindung folgende Formel hat:

   (I) $R_1\text{-CH=CH-}(CH_2)_n\text{-}R_2$ ;

   - die zweite ungesättigte Verbindung folgende Formel hat:

   (II) $R_3\text{-CH=CH-}R_4$;

   - das ungesättigte Produkt folgende Formel hat:

   (III) $R_4\text{-CH=CH-}(CH_2)_n\text{-}R_2$;

   - das ungesättigte Coprodukt folgende Formel hat:

   (IV) $R_2\ (CH_2)_n\text{-CH=CH-}(CH_2)_n\text{-}R_2$;

   wobei $R_1$ ein Wasserstoffatom oder einen Alkyl- oder Alkenylrest mit 1 bis 8 Kohlenstoffatomen darstellt; $R_2$ für $COOR_5$ oder CN oder CHO oder $CH_2OH$ oder $CH_2Cl$ oder $CH_2Br$ steht; $R_3$ und $R_4$ jeweils ein Wasserstoffatom oder einen Alkylrest mit 1 bis 4 Kohlenstoffatomen oder $COOR_5$ oder CN oder CHO oder $CH_2OH$ oder $CH_2Cl$ oder $CH_2Br$ darstellen, wobei $R_3$ und $R_4$ gleich oder verschieden sind und insgesamt nicht mehr als 6 Kohlenstoffatome enthalten; $R_5$ ein Wasserstoffatom oder einen Alkylrest mit 1 bis 4 Kohlenstoffatomen darstellt; und n eine ganze Zahl von 4 bis 11 ist.

**5.** Verfahren nach einem der Ansprüche 1 bis 4, wobei die zweite ungesättigte Verbindung ein Acrylat oder vorzugsweise Acrylnitril ist, die erste ungesättigte Verbindung eine Säure, ein ungesättigtes Nitril oder ein ungesättigter Ester, vorzugsweise ausgewählt aus Methyl-9-decenoat, 9-Decennitril, 10-Undecennitril und Methyl-10-undecenoat, ist, das ungesättigte Produkt ein Esternitril, ein Säurenitril, ein Dinitril oder ein ungesättigter Diester ist und das ungesättigte Coprodukt ein Diester, ein Dinitril oder eine ungesättigte Disäure ist.

**6.** Verfahren nach einem der Ansprüche 1 bis 5, wobei die Metathesereaktionen in flüssiger Phase, gegebenenfalls in einem Lösungsmittel, durchgeführt werden und vorzugsweise zur Herstellung von mindestens einer ungesättigten Verbindung in Gasform, ganz besonders bevorzugt Ethylen, im Reaktor führen, wobei das Verfahren deren kontinuierliche Entfernung aus dem Reaktor umfasst.

**7.** Verfahren nach einem der Ansprüche 1 bis 6, wobei der Umwandlungsgrad der ersten ungesättigten Verbindung am Ende der Produktionsphase 50 bis 98%, vorzugsweise 60-95%, ganz besonders bevorzugt 70 bis 90% beträgt.

**8.** Verfahren nach einem der Ansprüche 1 bis 7, das eine vorherige Phase der Auswahl des ersten Katalysators und des zweiten Katalysators aus einer Gruppe möglicher Katalysatoren vor der Produktionsphase umfasst.

**9.** Verfahren nach Anspruch 8, wobei die vorherige Auswahlphase Folgendes umfasst:

- Durchführung eines Referenzverfahrens für jeden möglichen Katalysator, wobei jedes Referenzverfahren das Beschicken des Reaktors mit der ersten ungesättigten Verbindung, das Beschicken des Reaktors mit der zweiten ungesättigten Verbindung und das Beschicken des Reaktors mit dem Katalysator umfasst, wobei die Bedingungen von Temperatur, Druck, Stöchiometrie und Konzentrationen der Reagenzien für jedes Referenzverfahren identisch zu den für die Produktionsphase verwendeten sind;
- Sammlung der Ergebnisse der Referenzverfahren, umfassend das Messen der Konzentrationen der chemischen Spezies in dem Reaktor im Zeitverlauf;
- Auswahl des ersten Katalysators und des zweiten Katalysators in Abhängigkeit von den Ergebnissen der Referenzverfahren.

**10.** Verfahren nach Anspruch 9, wobei der erste Katalysator der Katalysator ist, der im Referenzverfahren die maximale Wechselzahl bei dem gleichen Ziel-Umwandlungsgrad der ersten ungesättigten Verbindung aufweist.

**11.** Verfahren nach Anspruch 9 oder 10, wobei der zweite Katalysator ein Katalysator ist, für den im Referenzverfahren der Absolutwert der Ableitung der Reaktionsausbeute an ungesättigtem Coprodukt in Abhängigkeit von dem Umwandlungsgrad der ersten ungesättigten Verbindung, bestimmt bei einem Umwandlungsgrad von 1, minimal ist.

**12.** Verfahren nach Anspruch 11, wobei der zweite Katalysator aus den Katalysatoren, für die im Referenzverfahren der Absolutwert der Ableitung der Reaktionsausbeute an ungesättigtem Coprodukt in Abhängigkeit vom Umwandlungsgrad der ersten ungesättigten Verbindung, gemessen bei einem Umwandlungsgrad von 1, minimal ist, als der Katalysator ausgewählt wird, für den die Wechselzahl im Referenzverfahren für den gleichen Ziel-Umwandlungsgrad der ersten ungesättigten Verbindung maximal ist.

**13.** Verfahren nach Anspruch 9 oder 10, wobei:

- man zu unterschiedlichen Zeitpunkten jedes Referenzverfahrens die Zahl X berechnet, die durch die folgende Formel definiert ist:

$$(1) \quad X = (TTU_2/2)\times(z/C_0-TTU_1)/((1-TTG)\times TTU_1),$$

wobei $C_0$ die Anzahl Mole der ersten ungesättigten Verbindung im Reaktor vor Beginn der Beschickung mit Katalysator darstellt, TTG den Umwandlungsgrad der ersten ungesättigten Verbindung zum betreffenden Zeitpunkt darstellt, $TTU_1$ die Reaktionsausbeute an ungesättigtem Produkt zum betreffenden Zeitpunkt darstellt, $TTU_2$ die Reaktionsausbeute an ungesättigtem Coprodukt zum betreffenden Zeitpunkt darstellt und z die kumulative Gesamtanzahl der Mole der zweiten ungesättigten Verbindung, die in den Reaktor zum betreffenden Zeitpunkt eingebracht sind, darstellt;
- man zu verschiedenen Zeitpunkten jedes Referenzverfahrens die Zahl Y berechnet, die durch die folgende

Formel definiert ist:

$$(2)\quad Y = (p\times\alpha/(2+p))\times(z/(C_0\times TTU_1)-1) + (2+2p)\times(1-TTG)/((2+p)\times TTU_1),$$

wobei $C_0$, TTG, $TTU_1$ und z die vorstehende Bedeutung haben, p den Absolutwert der Ableitung der Funktion $TTU_2$(TTG) für einen Umwandlungsgrad gleich 1 darstellt und $\alpha$ den Wert $(2a/b)\times(C_0/C'_0)$ hat, a die Ableitung der Funktion $TTU_1$(TTG) für einen Umwandlungsgrad gleich 0 darstellt, b die Ableitung der Funktion $TTU_2$(TTG) für einen Umwandlungsgrad gleich 0 darstellt und $C'_0$ die Anzahl Mole der zweiten ungesättigten Verbindung im Reaktor vor Beginn der Beschickung mit Katalysator darstellt;
- man die Funktion Y(X) durch eine Gleichung $Y = \beta\times X$ oder vorzugsweise durch eine Gleichung $Y = \beta'\times X-\gamma'$ approximiert;
- man den zweiten Katalysator als den Katalysator auswählt, für den der Parameter $\beta$ oder $\beta'$ maximal ist.

**14.** Verfahren nach einem der Ansprüche 1 bis 13, wobei der Reaktor mit dem ersten Katalysator beschickt wird, bis der Umwandlungsgrad der ersten ungesättigten Verbindung einen Schwellenwert erreicht, wobei der Schwellenwert 30 bis 90%, vorzugsweise 40 bis 80% und ganz besonders 50 bis 70% beträgt.

**15.** Verfahren nach einem der Ansprüche 1 bis 14, wobei der Reaktor mit dem ersten Katalysator während einer Zeitdauer gleich oder vorzugsweise größer als eine(r) Schwellendauer beschickt wird, wobei die Schwellendauer diejenige ist, nach deren Ablauf der Umwandlungsgrad der ersten ungesättigten Verbindung $TTG_S$ und die Ausbeute an ungesättigtem Coprodukt $TTU_{2S}$ in einen Referenzverfahren durch folgende Gleichung $TTU_{2S} = TTU^*\times(TTG_S-1)/(TTG^*-1)$ verknüpft sind, wobei TTU* der Wert der am Ende der Produktionsphase gewünschten Ausbeute an ungesättigtem Coprodukt ist und TTG* der Wert des am Ende der Produktionsphase gewünschten Umwandlungsgrads der ersten ungesättigten Verbindung ist, wobei das Referenzverfahren die Beschickung des Reaktors mit der ersten ungesättigten Verbindung, die Beschickung des Reaktors mit der zweiten ungesättigten Verbindung und die Beschickung des Reaktors mit dem ersten Katalysator umfasst, wobei die Bedingungen von Temperatur, Druck, Stöchiometrie und Konzentrationen der Reagenzien für das Referenzverfahren identisch zu den während der Produktionsphase verwendeten sind.

**16.** Verfahren nach einem der Ansprüche 1 bis 15, wobei der Reaktor mit dem ersten Katalysator für eine Zeitdauer beschickt wird, die gleich oder vorzugsweise größer ist als eine Schwellendauer, die die Zeitdauer ist, nach der in einem Referenzverfahren die zweite Ableitung der Wechselzahl des ersten Katalysators in Abhängigkeit von der Zeit null wird, wobei das Referenzverfahren die Beschickung des Reaktors mit der ersten ungesättigten Verbindung, die Beschickung des Reaktors mit der zweiten ungesättigten Verbindung und die Beschickung des Reaktors mit dem ersten Katalysator umfasst, wobei die Bedingungen von Temperatur, Druck, Stöchiometrie und Konzentrationen der Reagenzien für das Referenzverfahren identisch zu den während der Produktionsphase verwendeten sind.

**17.** Verfahren nach einem der Ansprüche 1 bis 16, das vor der Produktionsphase eine vorherige Phase der Bestimmung der Dauer des Einbringens des ersten Katalysators umfasst, die Folgendes umfasst:

- Durchführung des Referenzverfahrens;
- Sammlung der Ergebnisse des Referenzverfahrens, umfassend das Messen der Konzentrationen der chemischen Spezies im Reaktor im Zeitverlauf; und
- Berechnung der Dauer des Einbringens des ersten Katalysators in Abhängigkeit von den Ergebnissen der Referenzverfahren.

**18.** Verfahren nach einem der Ansprüche 1 bis 17, wobei der erste Katalysator und/oder der zweite Katalysator Ruthenium-Carben-Katalysatoren sind und vorzugsweise aus den Katalysatoren der folgenden Formel (A) und (B) ausgewählt sind:

(A)

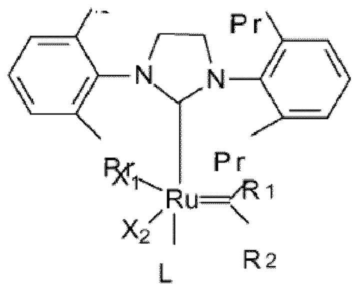

(B)

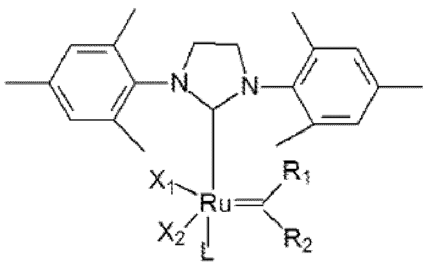

,

worin $X_1$ und $X_2$ anionische Liganden sind, L ein neutraler elektronenabgebender Ligand ist und $R_1$ und $R_2$ für H oder einen Substituenten mit 1 bis 20 Kohlenstoffatomen des Typs Alkyl, Alkenyl, Alkinyl, Aryl, Alkoxy, Alkenyloxy, Alkinyloxy, Aryloxy, Alkoxycarbonyl, Alkylthiol, Arylthiol, Alkylsulfonyl, Alkylsulfinyl ist; wobei der Substituent gegebenenfalls Gruppen des Typs Hydroxyl, Thiol, Thioether, Keton, Aldehyd, Ester, Ether, Amin, Imin, Amid, Nitro, Carbonsäure, Disulfid, Carbonat, Isocyanat, Carbodiimid, Carboalkoxy, Carbamat oder Halogen enthält, wobei $R_2$ vorzugsweise an L unter Bildung eines chelatbildenden Liganden gebunden ist.

**19.** Verfahren nach einem der Ansprüche 1 bis 18, wobei der erste Katalysator und/oder der zweite Katalysator aus den Katalysatoren der folgenden Formeln (A-1) bis (A-10) und (B-1) bis (B-5) ausgewählt sind:

(A-1)

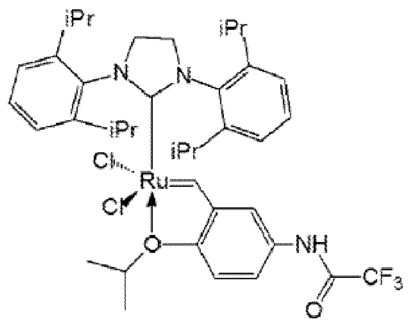

(A-2)

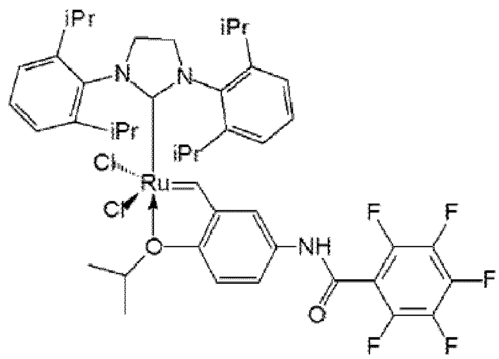

(A-3)

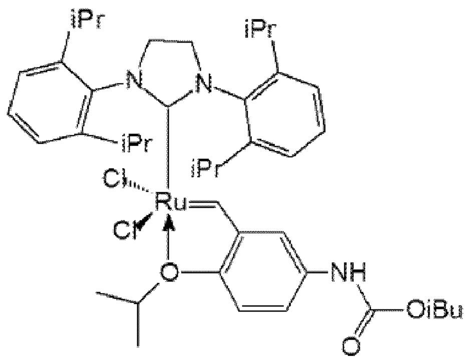

(A-4)

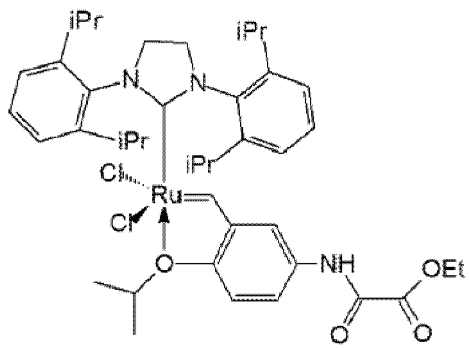

(A-5)

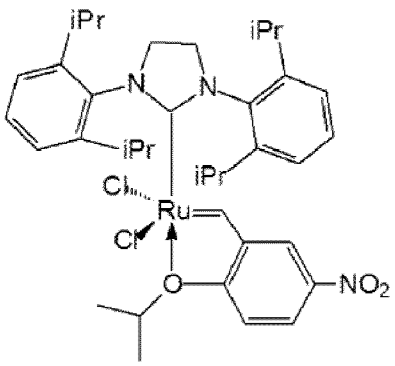

(A-6)

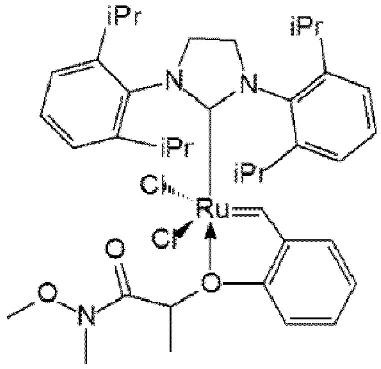

(A-7) 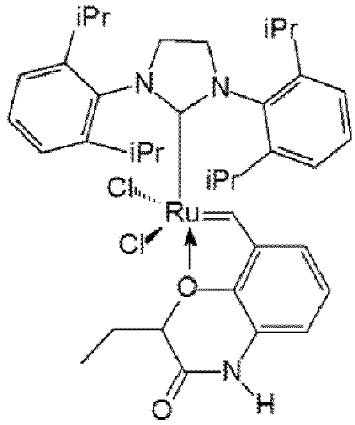

(A-8) 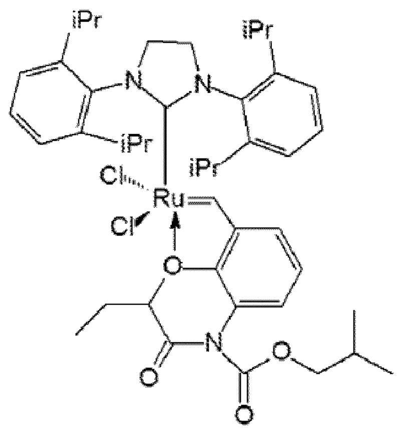

(A-9) 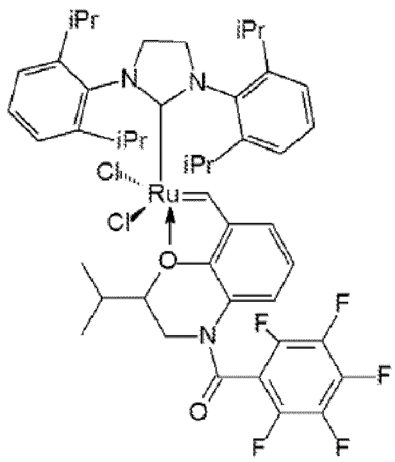

(A-10) 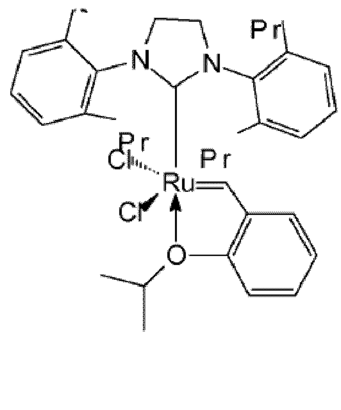

(B-1)

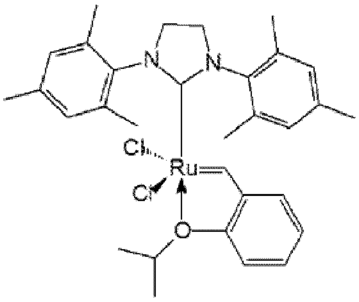

(B-2)

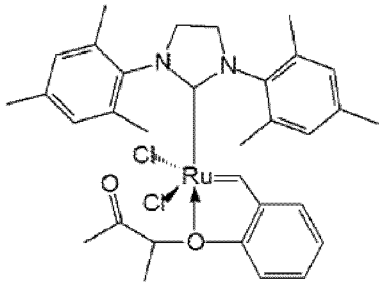

(B-3)

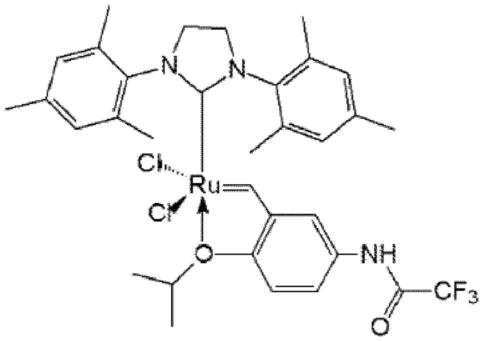

(B-4)

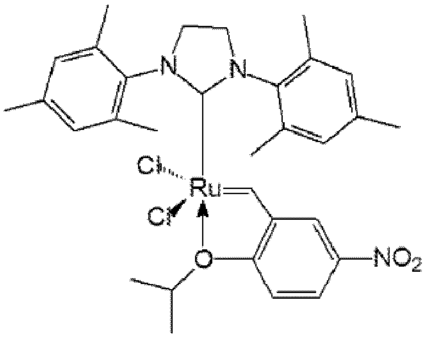

(B-5)

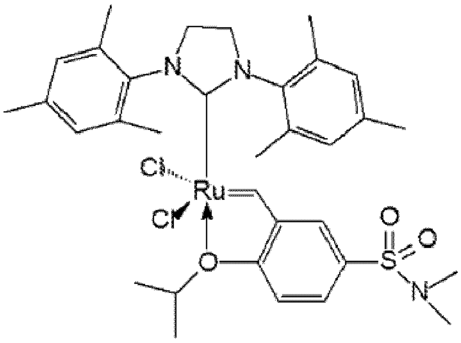

**20.** Verfahren nach Anspruch 18 oder 19, wobei der erste Katalysator ein Katalysator der Formel (A) ist und wobei der zweite Katalysator ein Katalysator der Formel (B) ist oder vorzugsweise wobei der erste Katalysator aus den Katalysatoren der Formeln (A-1) bis (A-10) ausgewählt ist und der zweite Katalysator aus den Katalysatoren der Formeln (B-1) bis (B-5) ausgewählt ist.

**21.** Syntheseverfahren zur Herstellung einer α,ω-Aminoalkansäure oder eines α,ω-Aminoalkansäureesters, umfassend die Synthese eines ungesättigten Produkts gemäß dem Verfahren nach einem der Ansprüche 1 bis 20, das ein ungesättigtes Esternitril oder Säurenitril ist, und eine Hydrierungsreaktion von diesem.

## Claims

**1.** A process for the synthesis of an unsaturated product by cross metathesis between a first unsaturated compound comprising at least 8 carbon atoms and a second unsaturated compound comprising less than 8 carbon atoms, the first unsaturated compound being capable of producing an unsaturated coproduct comprising more than 14 carbon atoms, by homometathesis, said process comprising at least one production phase which comprises:

- feeding a reactor with the first unsaturated compound;
- feeding the reactor with the second unsaturated compound;
- feeding the reactor with at least a first metathesis catalyst, then feeding the reactor with at least a second metathesis catalyst;
- withdrawing a product stream arising from the reactor;

the turnover number of the first catalyst being higher than the turnover number of the second catalyst so as to achieve the same target degree of conversion of the first unsaturated compound, the turnover numbers of the catalysts being determined under temperature, pressure, stoichiometry and reagent concentration conditions identical to those of the production phase.

**2.** The process as claimed in claim 1, wherein the target degree of conversion is from 30% to 95%, preferably from 40% to 90%, in particular from 50% to 80%, more particularly from 60% to 75%.

**3.** The process as claimed in claim 1 or 2, wherein the production phase is semi-batch, and preferably comprises successively:

(1) feeding the reactor with all of the first unsaturated compound and a first part of the second unsaturated compound, before starting up the reaction;
(2) gradually feeding the reactor with a second part of the second unsaturated compound and with the first catalyst;
(3) gradually feeding the reactor with a third part of the second unsaturated compound and with the second catalyst.

**4.** The process as claimed in one of claims 1 to 3, wherein:

- the first unsaturated compound has the formula:

(I) $R_1\text{-CH=CH-}(CH_2)_n\text{-}R_2$ ;

- the second unsaturated compound has the formula:

(II) $R_3\text{-CH=CH-}R_4$;

- the unsaturated product has the formula:

(III) $R_4\text{-CH=CH-}(CH_2)_n\text{-}R_2$;

- the unsaturated coproduct has the formula:

(IV) $R_2\text{-}(CH_2)_n\text{-CH=CH-}(CH_2)_n\text{-}R_2$ ;

$R_1$ representing a hydrogen atom or an alkyl or alkenyl radical comprising from 1 to 8 carbon atoms; $R_2$ representing $COOR_5$ or CN or CHO or $CH_2OH$ or $CH_2Cl$ or $CH_2Br$; $R_3$ and $R_4$ each representing a hydrogen atom or an alkyl radical comprising from 1 to 4 carbon atoms or $COOR_5$ or CN or CHO or $CH_2OH$ or $CH_2Cl$ or $CH_2Br$, $R_3$ and $R_4$ being identical or different and not comprising in total more than 6 carbon atoms; $R_5$ representing a hydrogen atom or an alkyl radical comprising from 1 to 4 carbon atoms; and n being an integer from 4 to 11.

**5.** The process as claimed in one of claims 1 to 4, wherein the second unsaturated compound is an acrylate or preferably acrylonitrile, the first unsaturated compound is an acid, an unsaturated nitrile or an unsaturated ester, preferably chosen from methyl 9-decenoate, 9-decenenitrile, 10-undecenenitrile and methyl 10-undecenoate, the unsaturated product is an unsaturated nitrile-ester, nitrile-acid, dinitrile or diester, and the unsaturated coproduct is an unsaturated diester, dinitrile or diacid.

**6.** The process as claimed in one of claims 1 to 5, wherein the metathesis reactions are carried out in the liquid phase, where appropriate in a solvent, and preferably result in the production of at least one unsaturated compound in gas form, more particularly preferably ethylene, in the reactor, the process comprising the continuous drawing off thereof from the reactor.

**7.** The process as claimed in one of claims 1 to 6, wherein the degree of conversion of the first unsaturated compound at the end of the production phase is from 50% to 98%, preferably from 60% to 95%, more particularly preferably from 70% to 90%.

**8.** The process as claimed in one of claims 1 to 7, comprising a preliminary phase of selecting the first catalyst and the second catalyst from a set of possible catalysts, before the production phase.

**9.** The process as claimed in claim 8, wherein the preliminary selection phase comprises:

- carrying out a reference process for each possible catalyst, each reference process comprising feeding the reactor with the first unsaturated compound, feeding the reactor with the second unsaturated compound and feeding the reactor with the catalyst, the temperature, pressure, stoichiometry and reagent concentration conditions for each reference process being identical to those used for the production phase;
- collecting the results of the reference processes, comprising measuring concentrations of chemical species in the reactor over time;
- choosing the first catalyst and the second catalyst according to the results of the reference processes.

**10.** The process as claimed in claim 9, wherein the first catalyst is the catalyst which has the maximum turnover number in the reference process, for the same target degree of conversion of the first unsaturated compound.

**11.** The process as claimed in claim 9 or 10, wherein the second catalyst is a catalyst for which, in the reference process, the absolute value of the derivative of the yield of the reaction in terms of unsaturated coproduct, as a function of the degree of conversion of the first unsaturated compound, determined at the degree of conversion of 1, is minimal.

**12.** The process as claimed in claim 11, wherein the second catalyst is chosen, from the catalysts for which, in the reference process, the absolute value of the derivative of the yield of the reaction in terms of unsaturated coproduct, as a function of the degree of conversion of the first unsaturated compound, measured at the degree of conversion of 1, is minimal, as being the catalyst for which the turnover number in the reference process is at a maximum, for the same target degree of conversion of the first unsaturated compound.

**13.** The process as claimed in claim 9 or 10, wherein:

- the number X is calculated at various instants of each reference process, said number X being defined by the following formula:

$$(1) \qquad X=(UDC_2/2)\times(z/C_0-UDC_1)/((1-ODC)\times UDC_1),$$

in which $C_0$ represents the number of moles of the first unsaturated compound in the reactor before the beginning of feeding with catalyst, ODC represents the degree of conversion of the first unsaturated compound at the instant under consideration, $UDC_1$ represents the yield of the reaction in terms of unsaturated product at the instant under consideration, $UDC_2$ represents the yield of the reaction in terms of unsaturated coproduct at the instant under consideration, and z represents the cumulative total number of moles of second unsaturated compound that are introduced into the reactor at the instant under consideration;

- the number Y is calculated at various instants of each reference process, said number Y being defined by the following formula:

$$(2) \qquad Y=(p\times\alpha/(2+p))\times(z/(C_0\times UDC_1)-1) + (2+2p)\times(1-ODC)/((2+p)\times UDC_1)$$

in which $C_0$, ODC, $UDC_1$ and z have the meaning above, p represents the absolute value of the derivative of the function $UDC_2(ODC)$ for a degree of conversion equal to 1, and $\alpha$ is $(2a/b)\times(C_0/C'_0)$, a representing the derivative of the function $UDC_1(ODC)$ for a degree of conversion equal to 0, b representing the derivative of the function $UDC_2(ODC)$ for a degree of conversion equal to 0 and $C'_0$ representing the number of moles of the second unsaturated compound in the reactor before the beginning of feeding with catalyst;

- the function Y(X) is approximated by an equation $Y=\beta\times X$ or, preferably, by an equation $Y=\beta'\times X-\gamma'$;
- the second catalyst is selected as being the catalyst for which the parameter $\beta$ or $\beta'$ is at a maximum.

**14.** The process as claimed in one of claims 1 to 13, wherein the reactor is fed with first catalyst until the degree of conversion of the first unsaturated compound reaches a threshold value, said threshold value being from 30% to 90%, preferably from 40% to 80% and more particularly from 50% to 70%.

**15.** The process as claimed in one of claims 1 to 14, wherein the reactor is fed with first catalyst for a duration equal to, or preferably greater than, a threshold duration, said threshold duration being that after which the degree of conversion of the first unsaturated compound $ODC_{TH}$ and the yield in terms of unsaturated coproduct $UDC_{2TH}$ in a reference process, are connected by the equation $UDC_{2TH}=UDC^*\times(ODC_{TH}-1)/(ODC^*-1)$, where UDC* is the value of the yield in terms of unsaturated coproduct that is desired at the end of the production phase, and UDC* is the value of the degree of conversion of the first unsaturated compound that is desired at the end of the production phase, the reference process comprising feeding the reactor with the first unsaturated compound, feeding the reactor with the second unsaturated compound and feeding the reactor with the first catalyst, the temperature, pressure, stoichiometry and reagent concentration conditions for the reference process being identical to those used during the production phase.

**16.** The process as claimed in one of claims 1 to 15, wherein the reactor is fed with first catalyst for a duration equal to, or preferably greater than, a threshold duration which is the duration after which, in a reference process, the second derivative of the turnover number of the first catalyst as a function of time is cancelled out, the reference process comprising feeding the reactor with the first unsaturated compound, feeding the reactor with the second unsaturated compound and feeding the reactor with the first catalyst, the temperature, pressure, stoichiometry and reagent concentration conditions for the reference process being identical to those used during the production phase.

**17.** The process as claimed in one of claims 1 to 16, comprising, before the production phase, a preliminary phase for determining the duration of introduction of the first catalyst, which comprises:

- carrying out the reference process;
- collecting the results of the reference process, comprising measuring concentrations of chemical species in the reactor over time; and
- calculating the duration of introduction of the first catalyst according to the results of the reference processes.

**18.** The process as claimed in one of claims 1 to 17, wherein the first catalyst and/or the second catalyst are ruthenium-carbene catalysts, and are preferably chosen from the catalysts of formulae (A) and (B) below:

(A) 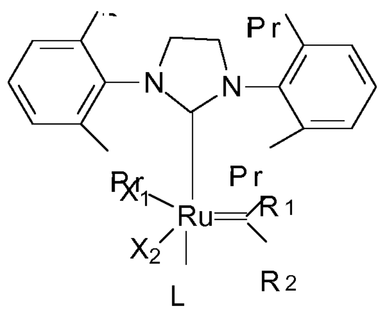

(B) 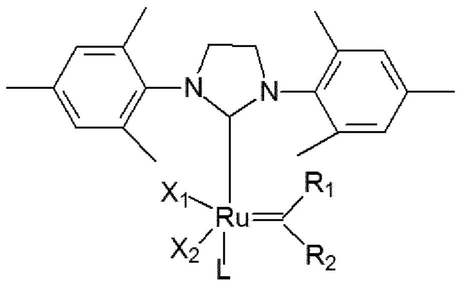

in which $X_1$ and $X_2$ are anionic ligands, L is an electron-donating neutral ligand, and $R_1$ and $R_2$ represent H or a substituent containing from 1 to 20 carbon atoms of alkyl, alkenyl, alkynyl, aryl, alkoxy, alkenyloxy, alkynyloxy, aryloxy, alkoxycarbonyl, alkylthiol, arylthiol, alkylsulfonyl or alkylsulfinyl type; the substituent optionally containing groups of hydroxyl, thiol, thioether, ketone, aldehyde, ester, ether, amine, imine, amide, nitro, carboxylic acid, disulfide, carbonate, isocyanate, carbodiimide, carboalkoxy, carbamate or halogen type, $R_2$ preferably being bonded to L so as to form a chelating ligand.

**19.** The process as claimed in one of claims 1 to 18, wherein the first catalyst and/or the second catalyst are chosen from the catalysts of formulae (A-1) to (A-10) and (B-1) to (B-5) below:

(A-1) 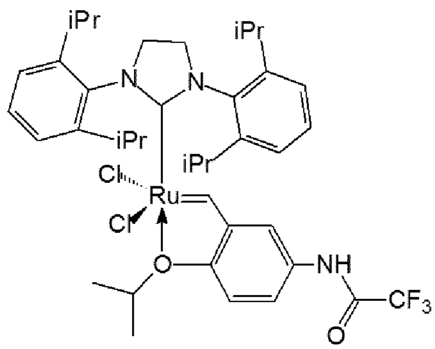

(A-2) 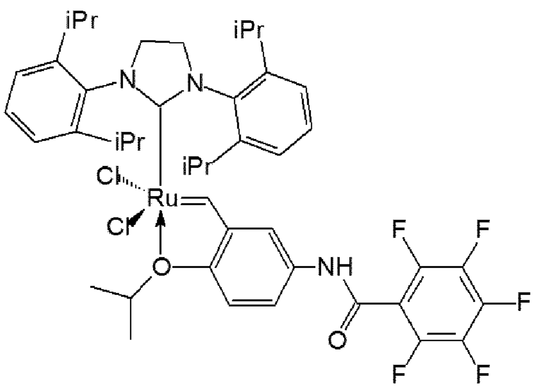

(A-3)

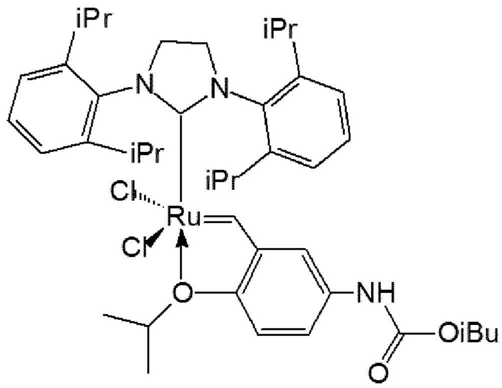

(A-4)

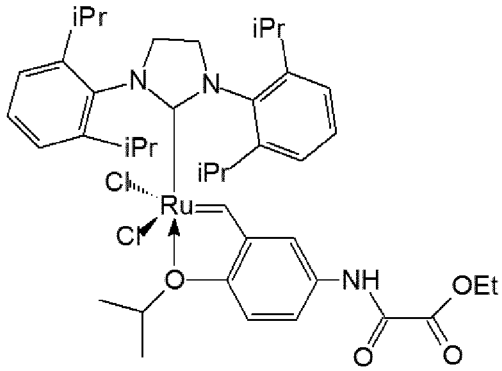

(A-5)

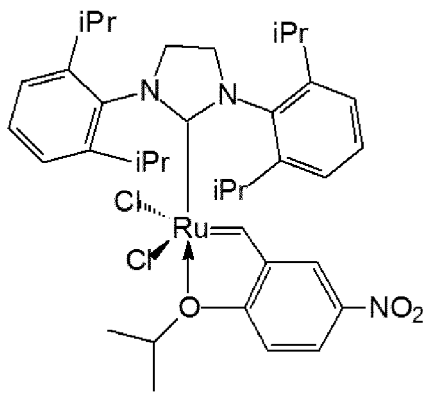

(A-6)

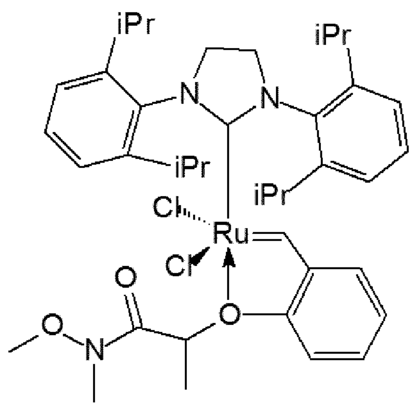

(A-7)

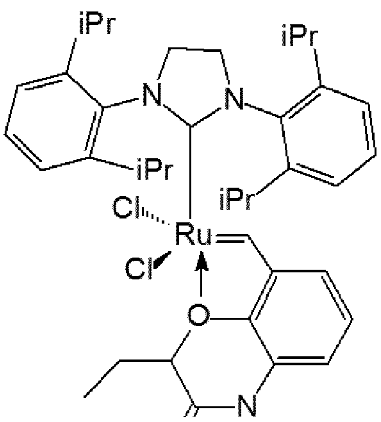

(A-8)

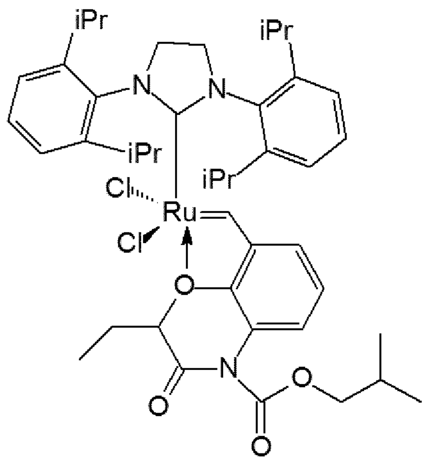

(A-9)

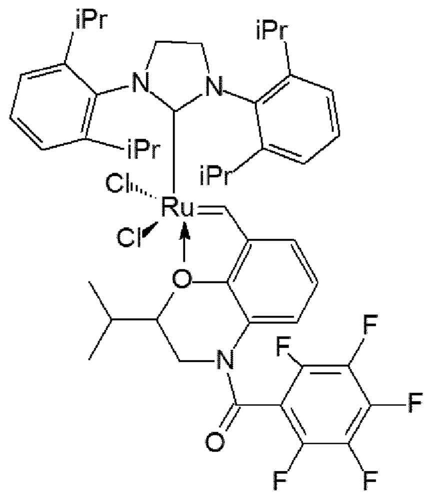

(A-10)

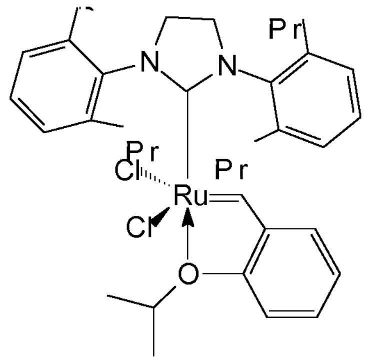

(B-1)

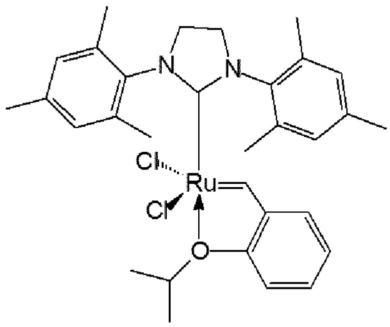

(B-2)

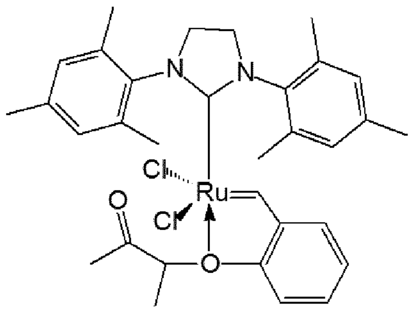

(B-3)

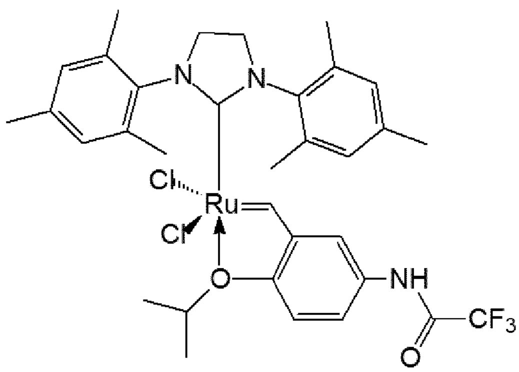

(B-4)

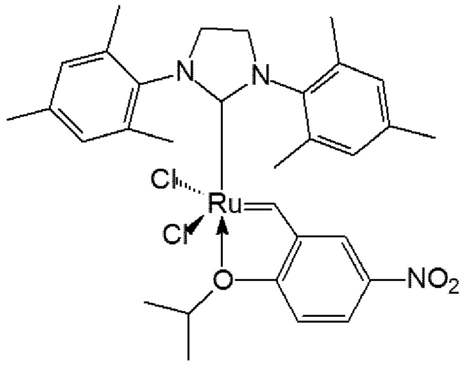

(B-5)

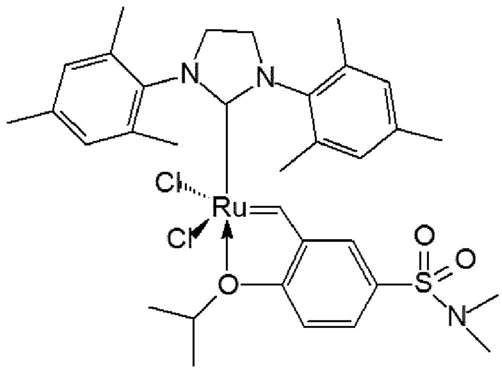

**20.** The process as claimed in claim 18 or 19, wherein the first catalyst is a catalyst of formula (A) and wherein the second catalyst is a catalyst of formula (B), or preferably wherein the first catalyst is chosen from the catalysts of formulae (A-1) to (A-10) and the second catalyst is chosen from the catalysts of formulae (B-1) to (B-5).

**21.** A process for the synthesis of an α,ω-aminoalkanoic acid or ester, comprising the synthesis of an unsaturated product according to the process of one of claims 1 to 20, which is an unsaturated nitrile-ester or nitrile-acid, and a reaction for hydrogenation thereof.

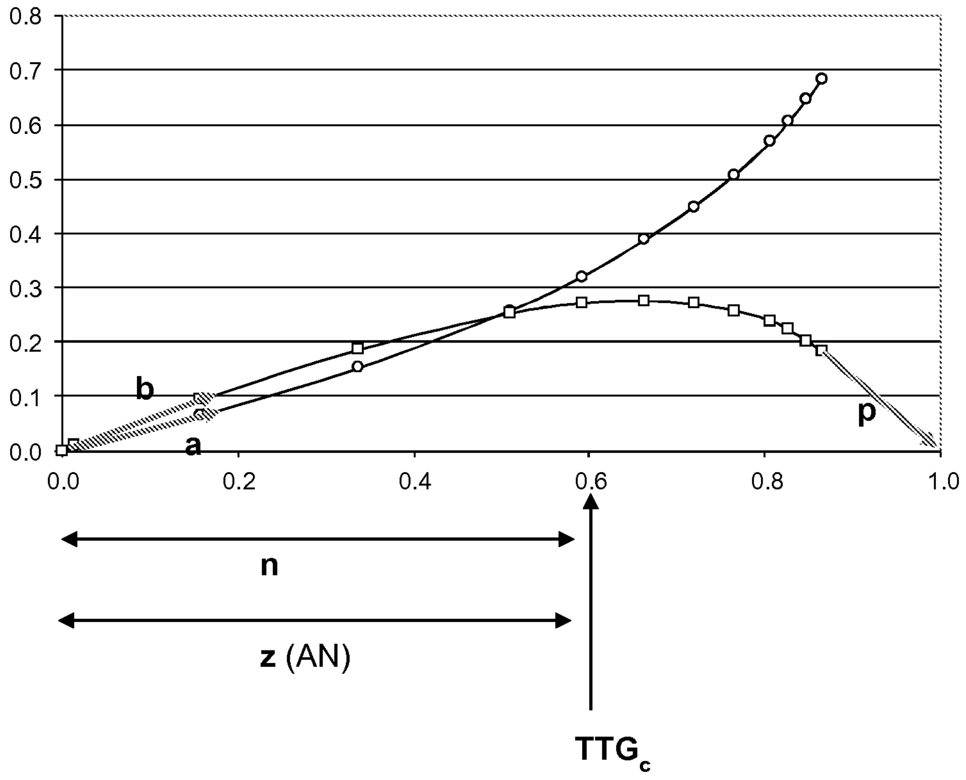
0.8
0.7
0.6
0.5
0.4
0.3
0.2
0.1
0.0
0.0
0.2
0.4
0.6
0.8
1.0
b
a
p
n
z (AN)
TTG$_c$

Fig. 1

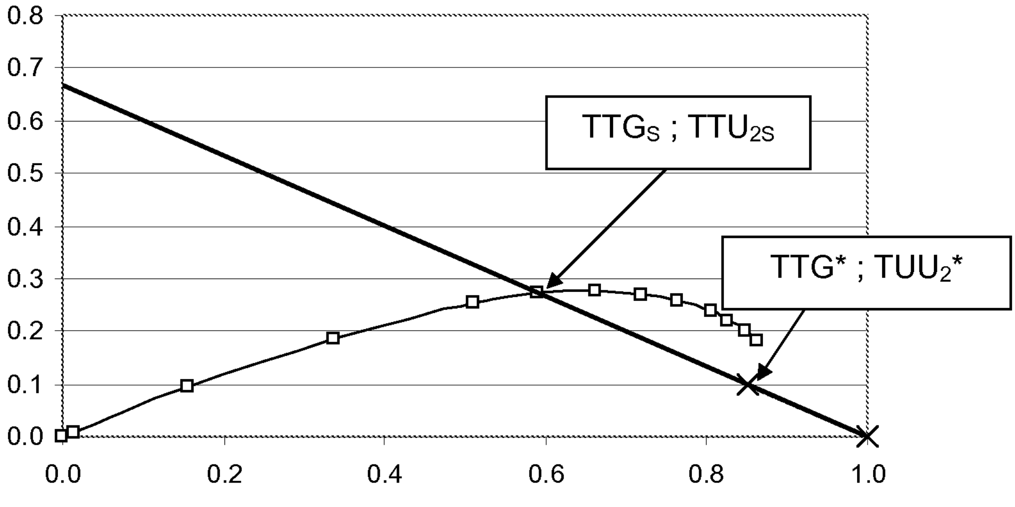
0.8
0.7
0.6
0.5
0.4
0.3
0.2
0.1
0.0
0.0
0.2
0.4
0.6
0.8
1.0
TTG$_S$ ; TTU$_{2S}$
TTG* ; TUU$_2$*

Fig. 2

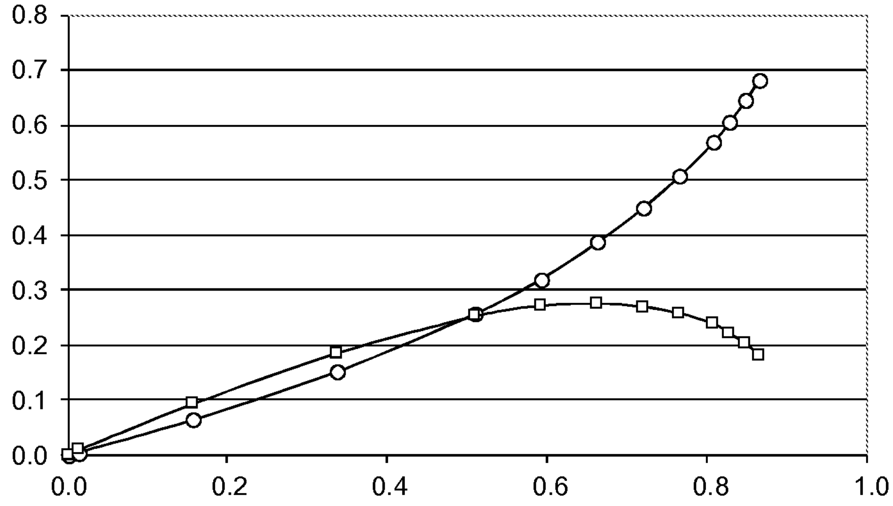
0.8
0.7
0.6
0.5
0.4
0.3
0.2
0.1
0.0
0.0
0.2
0.4
0.6
0.8
1.0

**Fig. 3**

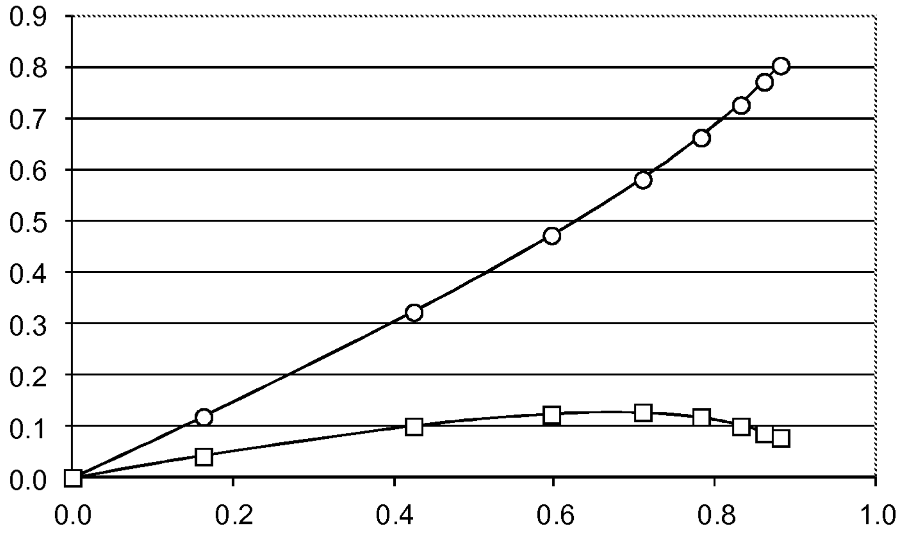
0.9
0.8
0.7
0.6
0.5
0.4
0.3
0.2
0.1
0.0
0.0
0.2
0.4
0.6
0.8
1.0

**Fig. 4**

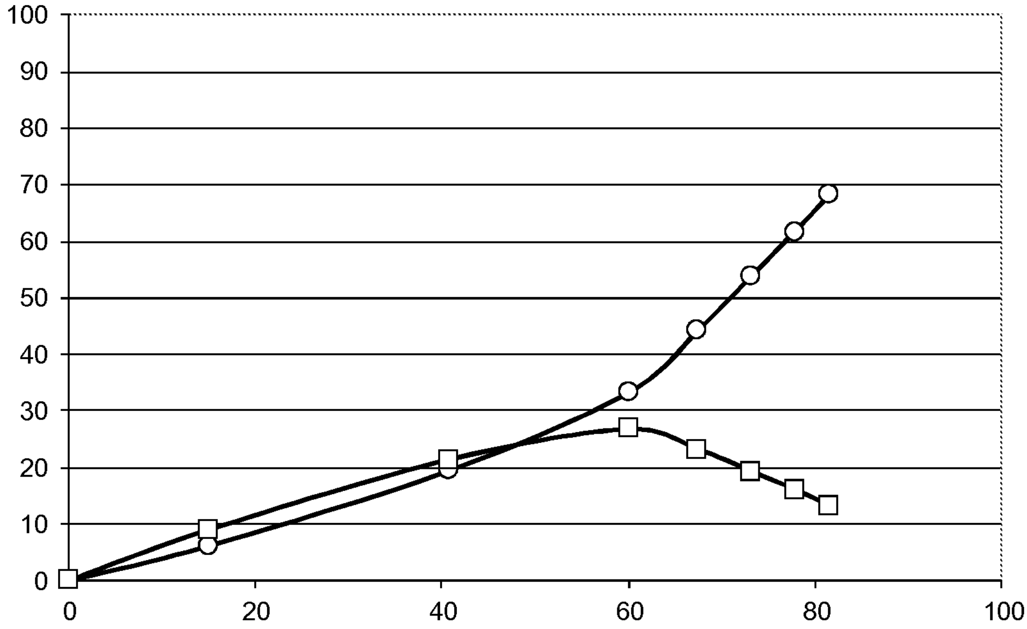
100
90
80
70
60
50
40
30
20
10
0
0
20
40
60
80
100

Fig.5

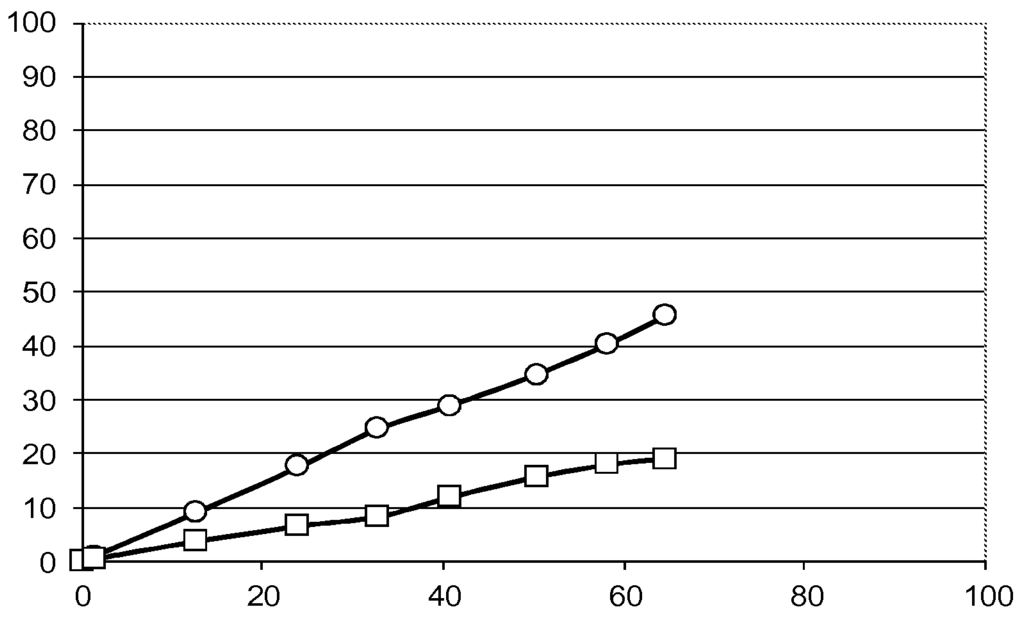
100
90
80
70
60
50
40
30
20
10
0
0
20
40
60
80
100

Fig. 6

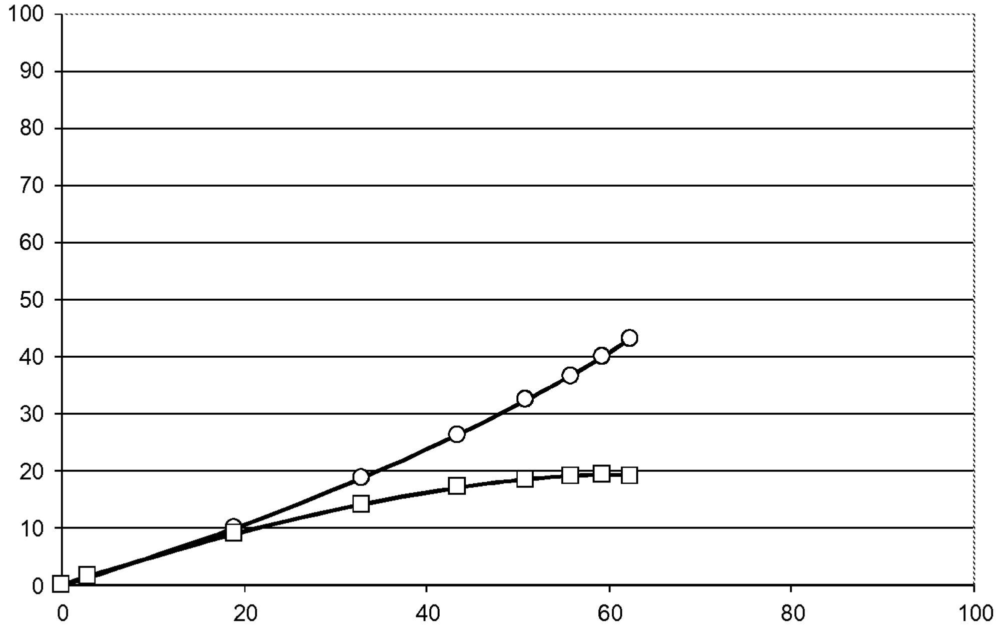


Fig. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2912741
- FR 2938533
- FR 2941694
- FR 2959742 A
- US 20110113679 A


### Littérature non-brevet citée dans la description


- **SCHROCK et al.** *J. Am. Chem. Soc.,* 1986, vol. 108, 2771
- **BASSET et al.** *Angew. Chem., Ed. Engl.,* 1992, vol. 31, 628
- **GRUBBS et al.** *Angew. Chem., Ed. Engl.,* 1995, vol. 34, 2039
- *Organic Letters,* 1999, vol. 1, 953
- *Aldrichimica Acta,* 2007, vol. 40 (2), 45-52